# EUROPEAN PATENT SPECIFICATION

(11) **EP 4 479 128 B1**
(45) Date of publication and mention of the grant of the patent: **19.08.2026**
(21) Application number: 23711143.0
(22) Date of filing: 16.02.2023
(51) Int. Cl.: A61N 1/05, A61B 5/304, A61B 5/00, A61N 1/36, A61B 5/293, A61B 5/294, A61B 5/31, A61B 5/37, A61B 5/388

(54) **A MULTICHANNEL VERSATILE BRAIN ACTIVITY CLASSIFICATION AND CLOSED LOOP NEUROMODULATION SYSTEM, DEVICE AND METHOD USING A HIGHLY MULTIPLEXED MIXED-SIGNAL FRONT-END**
MEHRKANALIGES VIELSEITIGES GEHIRNAKTIVITÄTSKLASSIFIZIERUNGS- UND REGELKREISNEUROMODULATIONSSYSTEM, VORRICHTUNG UND VERFAHREN MIT VERWENDUNG EINES HOCHMULTIPLEXIERTEN MISCHSIGNAL-FRONTENDS
SYSTÈME, DISPOSITIF ET PROCÉDÉ DE CLASSIFICATION D'ACTIVITÉ CÉRÉBRALE POLYVALENTE MULTICANAL ET DE NEUROMODULATION EN BOUCLE FERMÉE UTILISANT UN SIGNAL MIXTE FRONTAL HAUTEMENT MULTIPLEXÉ

(30) Priority: 17.02.2022 US 202263311090 P
(43) Date of publication of application: 25.12.2024
(60) Divisional application: 26159177.0 / 4 740 861
(73) Proprietor: Ecole Polytechnique Fédérale de Lausanne (EPFL), 1015 Lausanne (CH)
(72) Inventor: SHOARAN, Mahsa, 1025 St-Sulpice (CH); SHIN, Uisub, 1202 Genève (CH); ZHU, Bingzhao, 1202 Genève (CH)
(74) Representative: reuteler & cie SA
(86) International application number: PCT/IB2023/051413
(87) International publication number: WO 2023/156932

(56) References cited:
- WO-A1-2013/123358
- US-A1- 2009 082 691
- US-A1- 2011 193 623
- US-A1- 2016 249 846
- US-B2- 8 937 509

## Description

### FIELD OF THE INVENTION

The present invention is directed to the field of closed-loop neuromodulation.

### BACKGROUND

Closed-loop neuromodulation can alleviate disease symptoms and provide sensory feedback in various neurological disorders and injuries. Energy-efficient realization of closed-loop devices with on-site classification is critical to enhancing therapeutic efficacy. Despite recent advances, existing devices, for example system-on-chip devices (SoCs), with integrated machine learning are constrained by low channel count, for example a channel count around 8-32, and poor generalizability. In light of these deficiencies of the state of the art, strongly improved methods, systems and devices for closed-loop neurostimulation are strongly desired, to provide for high channel count, low power consumption, reduced surface area usage for chip implementation, and for providing superior performance and a multitude of applications fields over the state of the art.

US 2016/249846 A1 relates to a multichannel patient-specific scalable electroencephalography (EEG) acquisition SoC.

WO 2013/123358 A1 relates to an integrated circuit chip and method for EEG monitoring. US 2009/082691 A1 relates to a frequency selective monitor and method.

US 2011/193623 A1 relates to a large time constant steering circuit and its applications in a slow steering current digital-to-analog converter, an instrumentation amplifier with DC servo-loop, and an EEG acquisition application-specific integrated circuit.

US 8 357 509 B2 relates to a multi-channel biopotential signal acquisition system.

### SUMMARY

The invention is set forth in claim 1. Dependent claims recite advantageous embodiments of the invention.

### BRIEF DESCRIPTION OF THE SEVERAL VIEWS OF THE DRAWINGS

The accompanying drawings, which are incorporated herein and constitute part of this specification, illustrate the presently preferred embodiments of the invention, and together with the general description given above and the detailed description given below, serve to explain features of the invention.

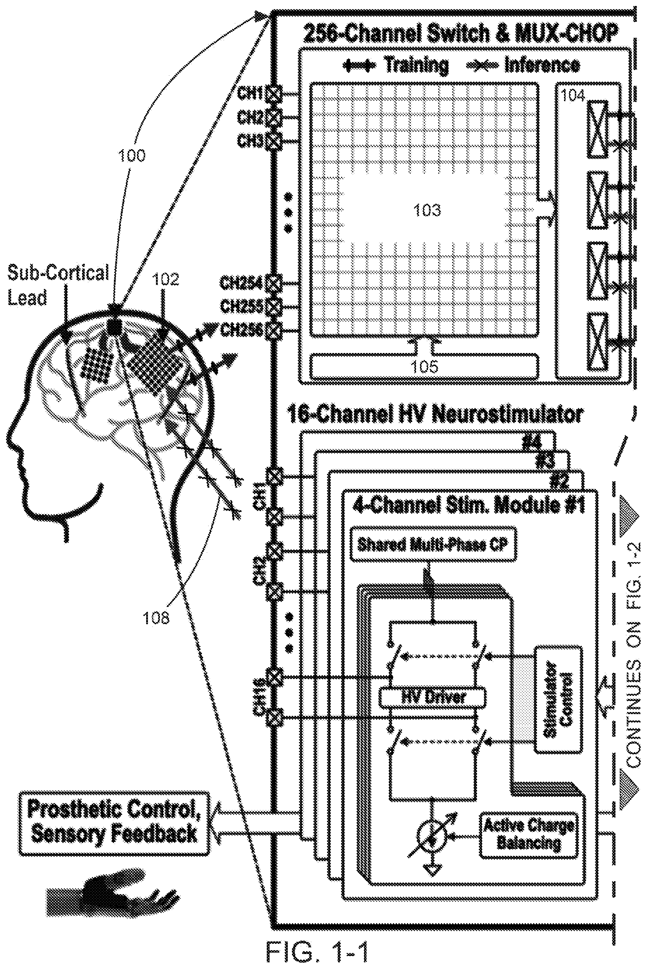

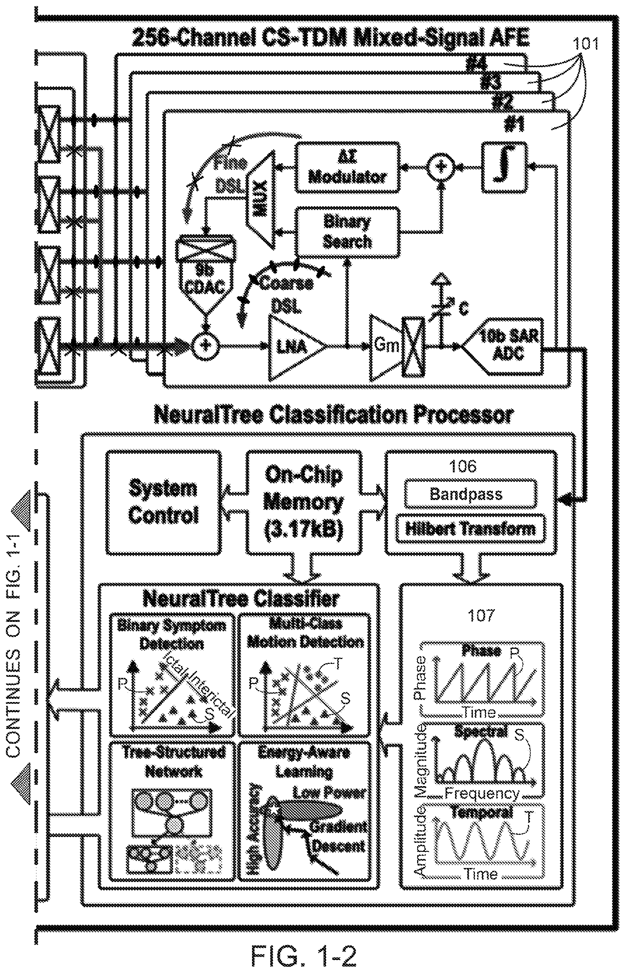

FIG. 1 depicts a schematic and exemplary overview of a system for versatile brain activity classification system-on-chip ("SoC") with high-density sensing and closed-loop neuromodulation, interconnected to an implantable matrix of electrodes (cortical grid) according to one aspect of the present invention;

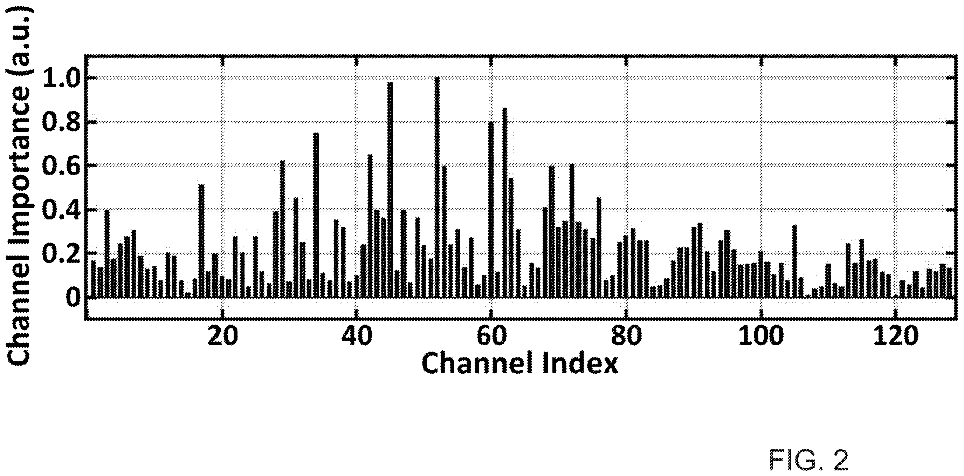

FIG. 2 contains a graph showing a relative importance of 128 iEEG channels from an epileptic patient and evaluated with 5-fold cross-validation;

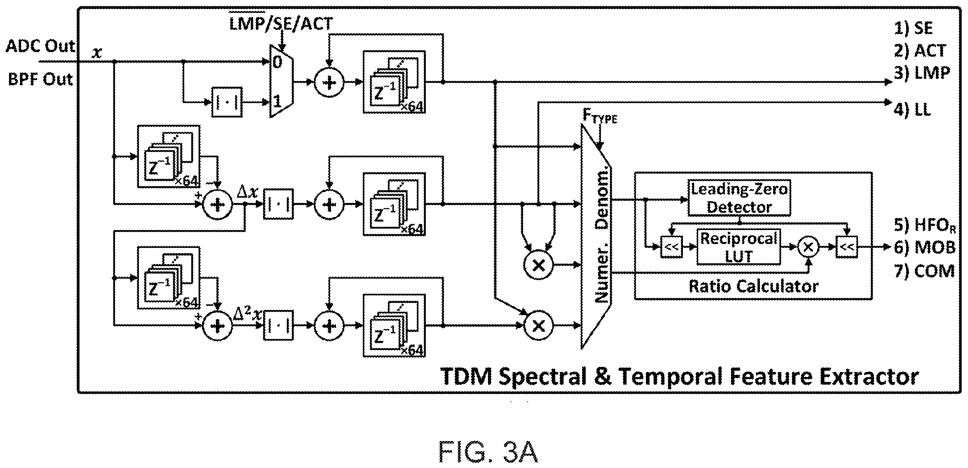

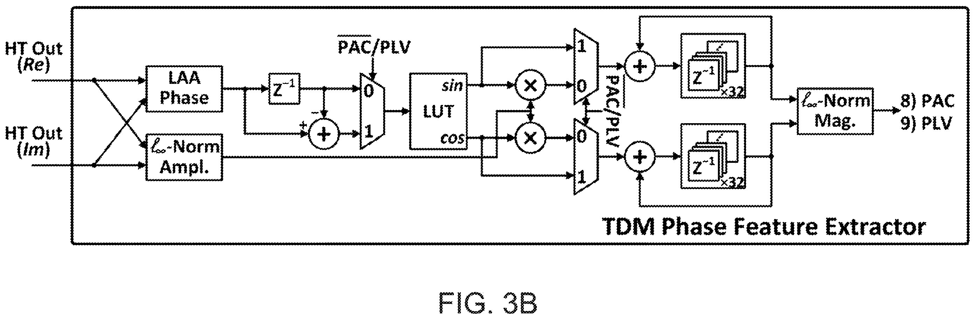

FIG. 3 shows hardware implementations of the TDM FEE: FIG. 3(a) contains a temporal and spectral feature extractor and FIG. 3(b) a phase feature extractor;

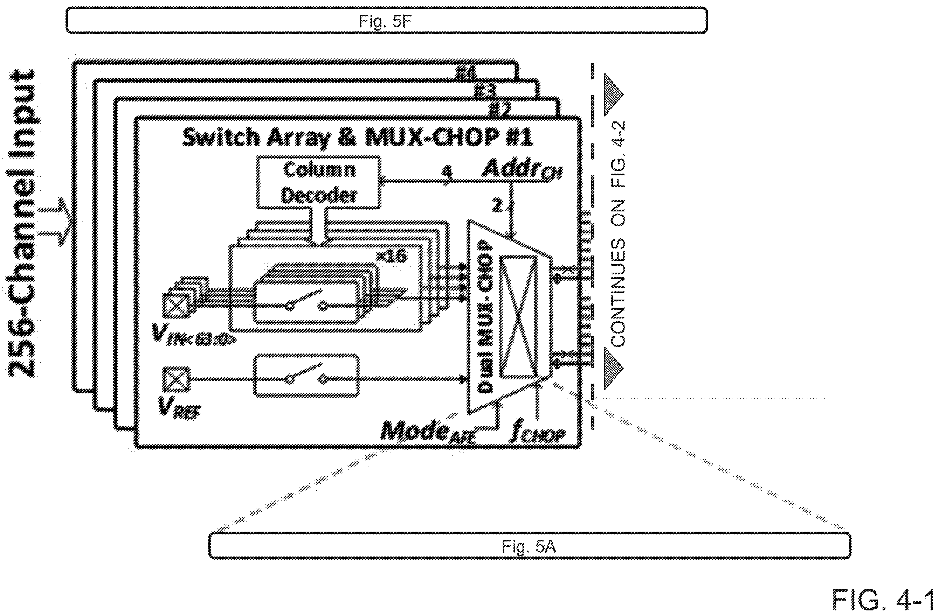

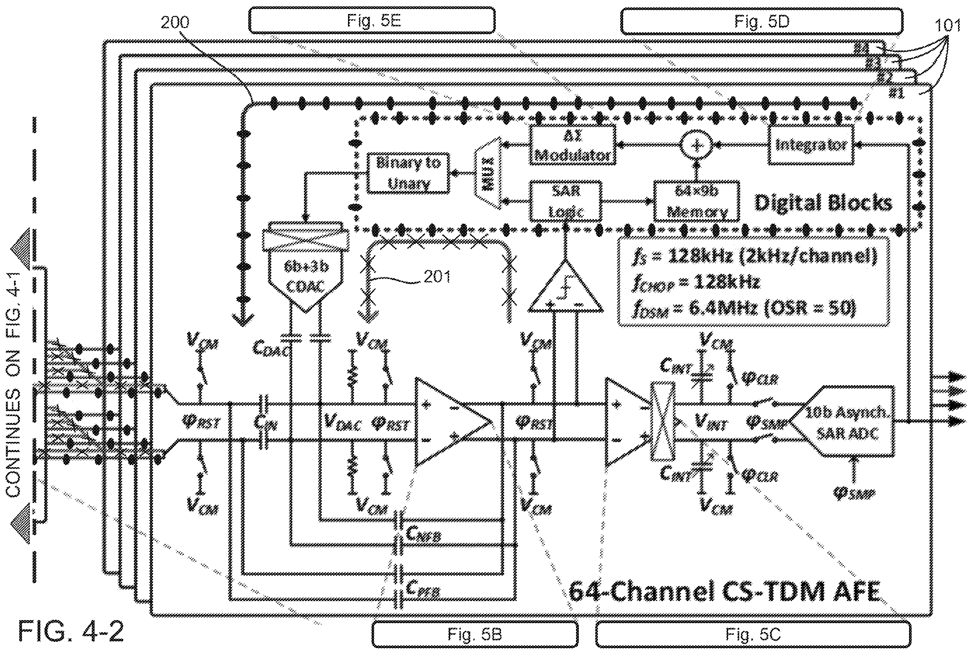

FIG. 4 depicts a chopper-stabilized time-division-multiplexed (CS-TDM) analog front-end device (AFE) having an exemplary number of 256 input channels operatively connected to the cortical grid, including a two-step fast-settling mixed-signal DC servo loop (DSL) having a coarse and fine servo loop, according to another aspect of the present invention;

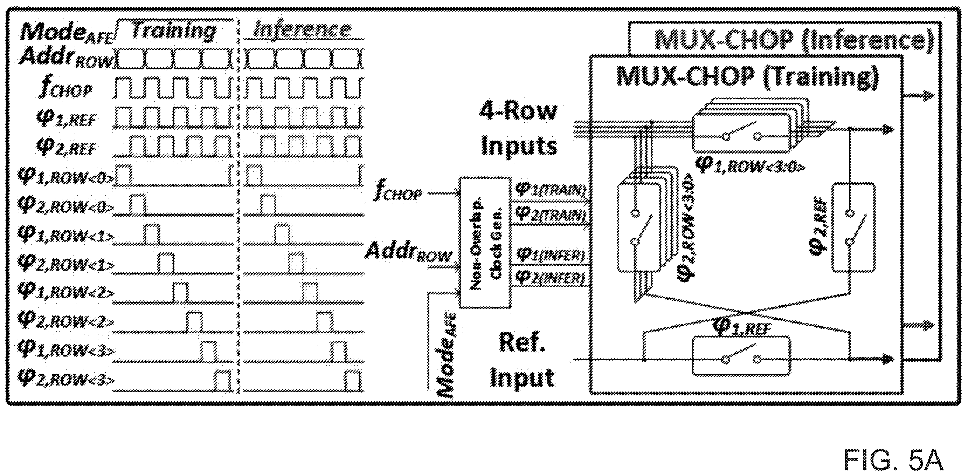

FIG. 5A shows an aspect of FIG. 4 showing a schematic and exemplary view of an exemplary 16×16 switch matrix and dual multiplexing choppers (MUX-CHOP) for configuring inputs in training and inference modes, the switch matrix operatively interconnected to the cortical grid;

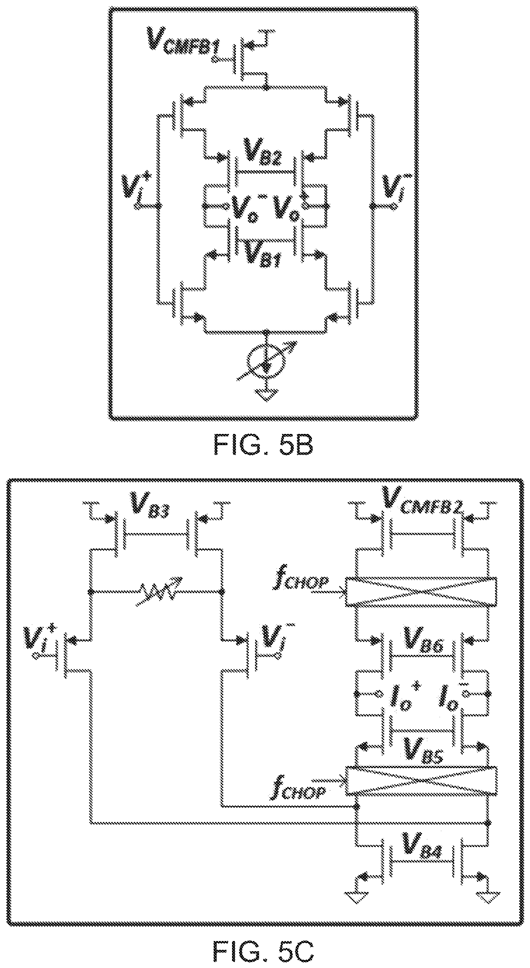

FIG. 5B shows an aspect of FIG. 4, showing an exemplary circuit for a differential amplifier in the AFE for low-noise amplification of input signals. The low-noise amplifier (LNA) is based on an exemplary current-reuse cascode amplifier to provide a high open-loop gain and improved noise efficiency, where by a capacitive feedback with the capacitors C_{IN}, C_{NFB} shown in FIG. 4, the LNA provides a low closed-loop gain and a high bandwidth to enable rapid offset cancellation in the coarse DSL, and the LNA receives input signals from the switch matrix and MUX-CHOP and sends out amplified signals to the dynamic comparator (in the coarse step) or to the Gₘ-C integrator (in the fine step);
FIG. 5C shows an aspect of FIG. 4, showing an exemplary circuit having transconductance amplifier in the AFE for additional signal amplification. The transconductance amplifier is based on a folded cascode architecture with source degeneration. The transconductance amplifier along with the capacitors C_{INT} (shown in FIG. 4) acts as a charge-sampling integrator, herein referred to as a Gₘ-C integrator, to perform anti-aliasing lowpass filtering prior to analog-to-digital conversion. The additional gain is programmable with the tunable source degeneration resistor and integration capacitors. The Gₘ-C integrator receives the output of the LNA and sends amplified and lowpass-filtered signals to the analog-to-digital converter for digitization;

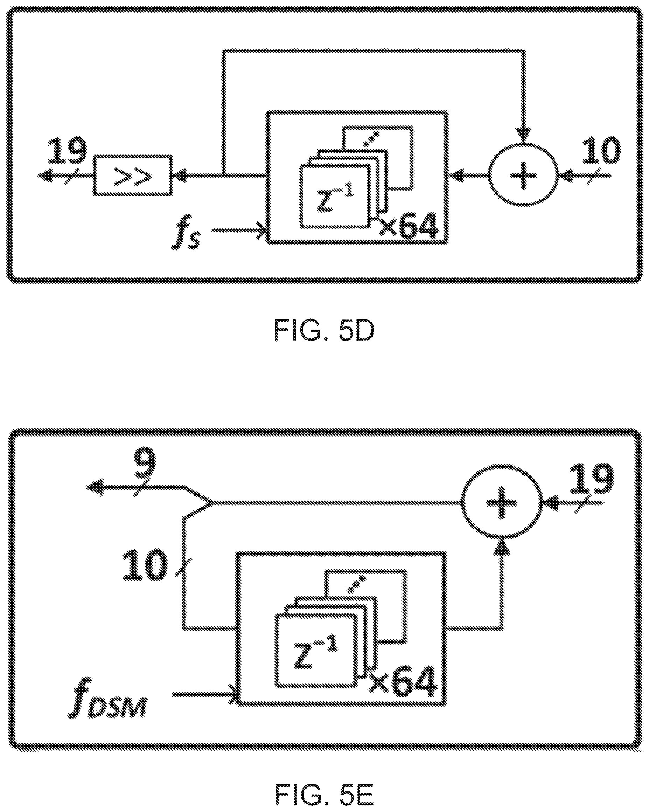

FIG. 5D shows an aspect of FIG. 4, showing an exemplary implementation of digital integrator for the signals provided by the analog-to-digital converter (ADC) as a part of the digital block of the AFE, where the integrator extracts from the ADC output undesired low-frequency signal components including residual EDOs after the coarse EDO cancellation;
FIG. 5E shows an aspect of FIG. 4, showing an exemplary implementation of digital delta sigma modulator as a part of the digital block of the AFE;

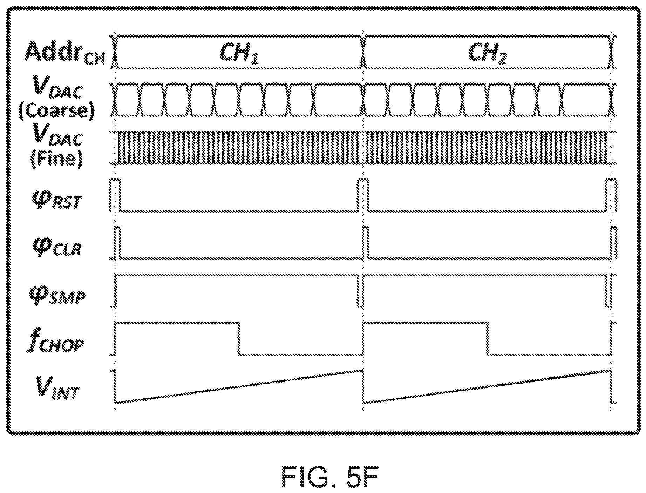

FIG. 5F shows an aspect of FIG. 4, depicting a timing graph of different signals of the AFE. The plurality of input channels are sequentially selected by a channel addressing signal (Addr_{CH}), and the two V_{DAC} signals show the change in the voltage output of the ADC in the coarse and fine EDO cancellation steps. Intermediates nodes in the AFE are periodically reset between two adjacent channels by two control signals (φ_{RST} and φ_{CLR}) to reduce inter-channel crosstalk. φ_{SMP} is a signal for controlling charge sampling in the Gₘ-C integrator, and V_{INT} plots charges integrated over a time during which φ_{SMP} is HIGH. f_{CHOP} is a signal for controlling choppers, represented by the rectangular cross boxes in FIG. 4;

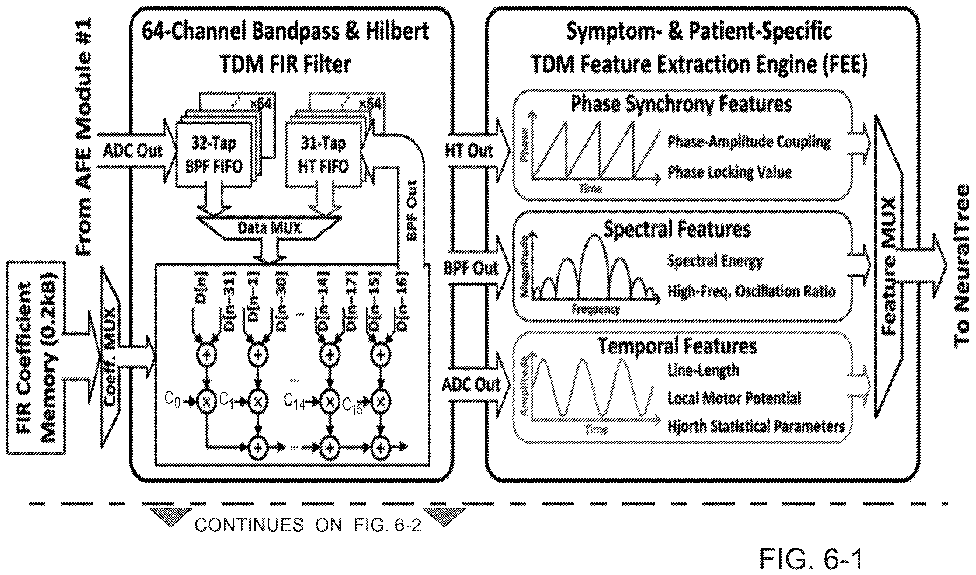

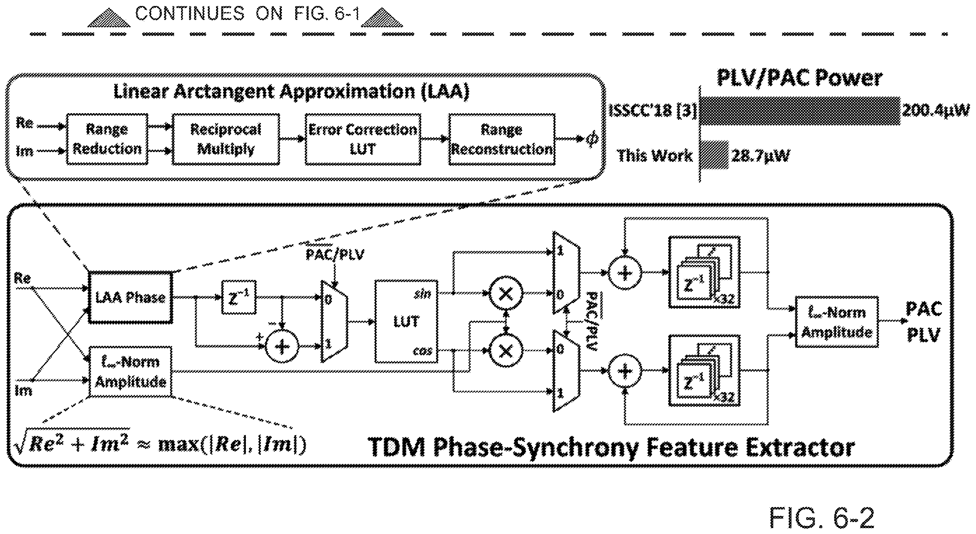

FIG. 6 shows different exemplary schematics of the time-division-multiplexed (TDM) finite impulse response (FIR) filter device receiving input signals from the AFE device of FIG. 4, the TDM feature extraction engine (FEE) having three different feature extractors including a phase-synchrony feature extractor, a frequency feature extractor, and a temporal feature extractor, and also showing a more detailed view in the lower section of the phase-synchrony feature extractor of the FEE including a linear arctangent approximation device (LAA), according to another aspect of the present invention;

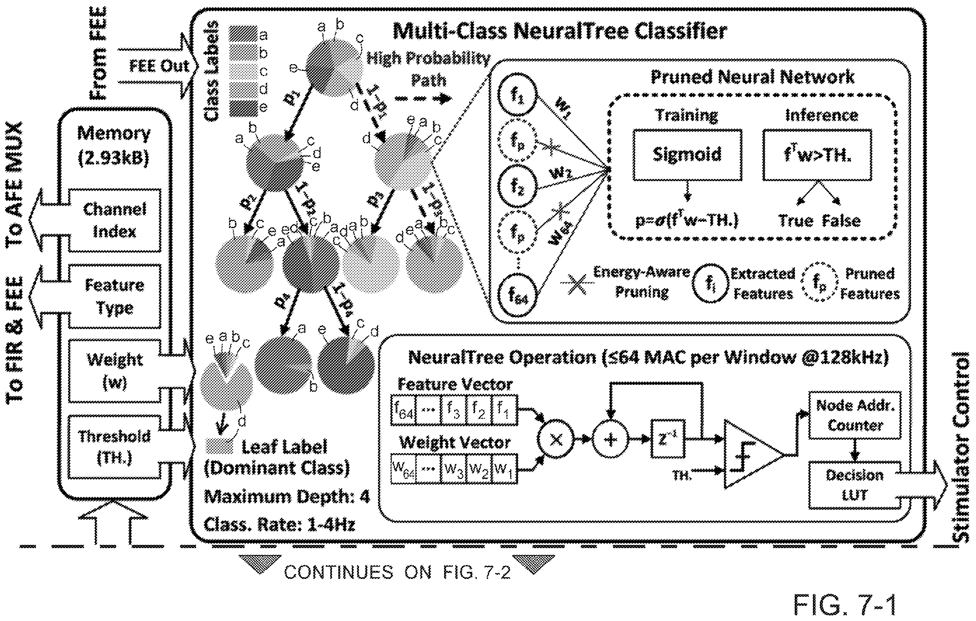

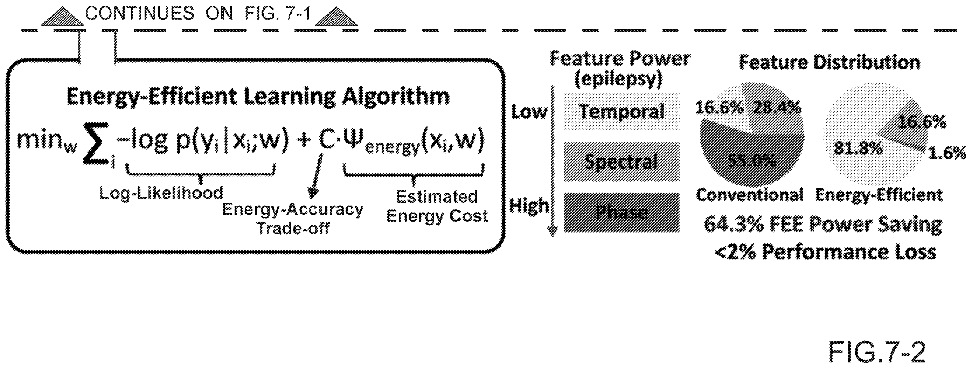

FIG. 7 shows a schematic and exemplary representation of the low-power tree-structured hierarchical neural network (NeuralTree) classifier, generating control signals for stimulating a brain of a patient or user with the plurality of implanted electrodes, for example the cortical grid or the sub-cortical lead, and having input signals from the FEE and weights and thresholds provided by an energy-efficient learning algorithm, the energy-efficient learning algorithm including a cost function minimization algorithm that allows to reduce the energy consumption as the cost factor, according to still one aspect of the present invention;

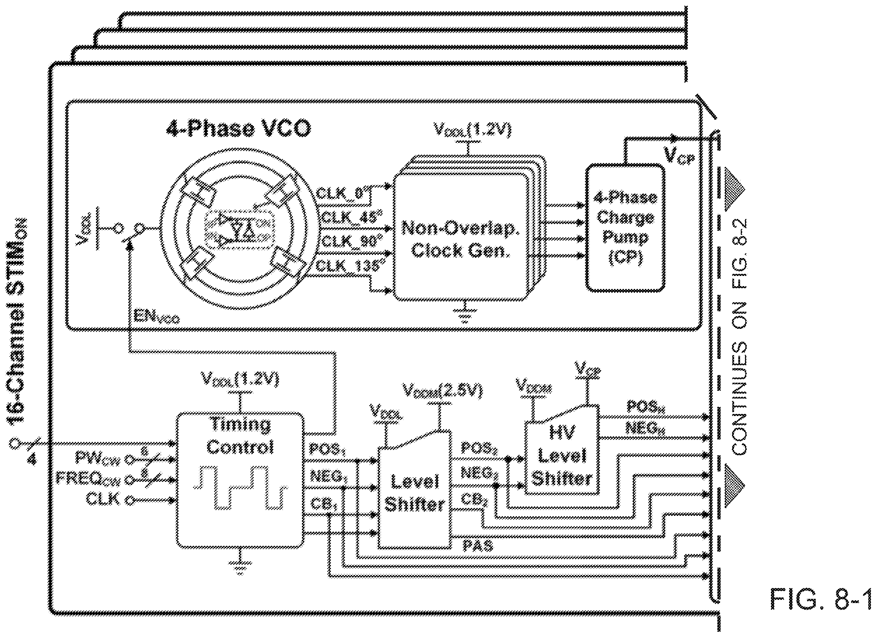

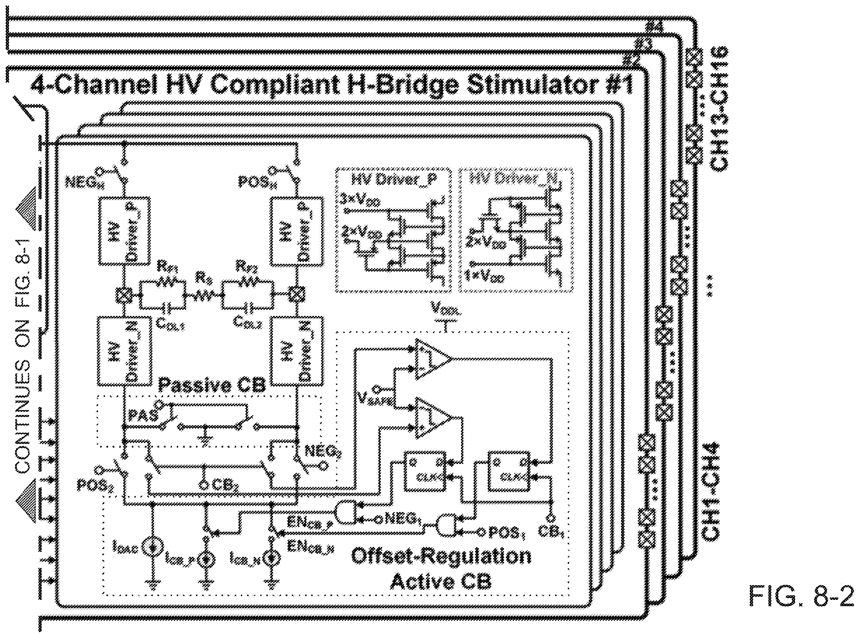

FIG. 8 showing a schematic and exemplary representation of a high-voltage (HV) compliant multi-channel neurostimulator for interconnection to the plurality of electrodes that can be implanted to a brain, using an exemplary number of 16 channels;

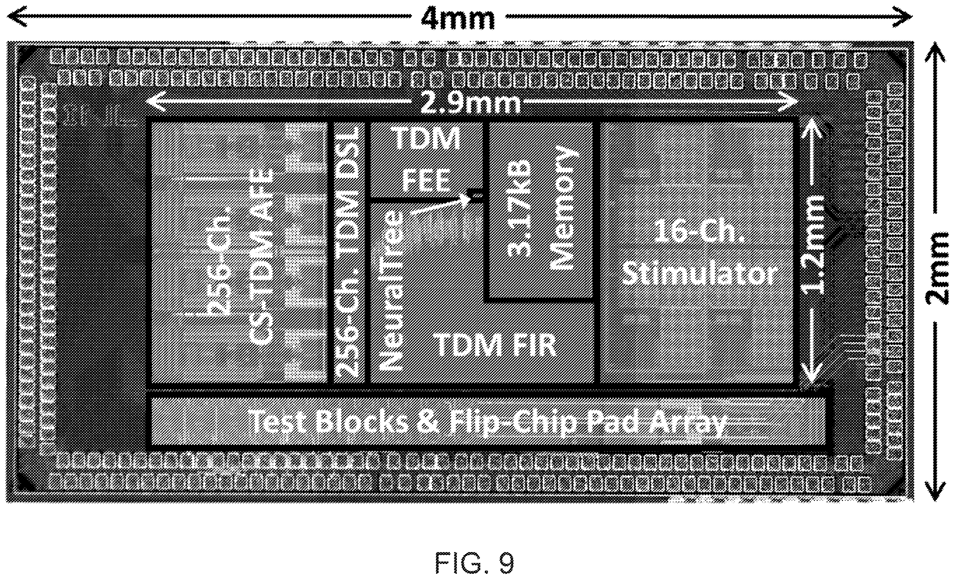

FIG. 9 shows an exemplary implementation of the device into a chip, including different surfaces areas used for the analog front-end device (AFE), the DC servo loop (DSL), the finite impulse response (FIR) filter, the feature extraction engine (FEE), the neurostimulator, and the memory;

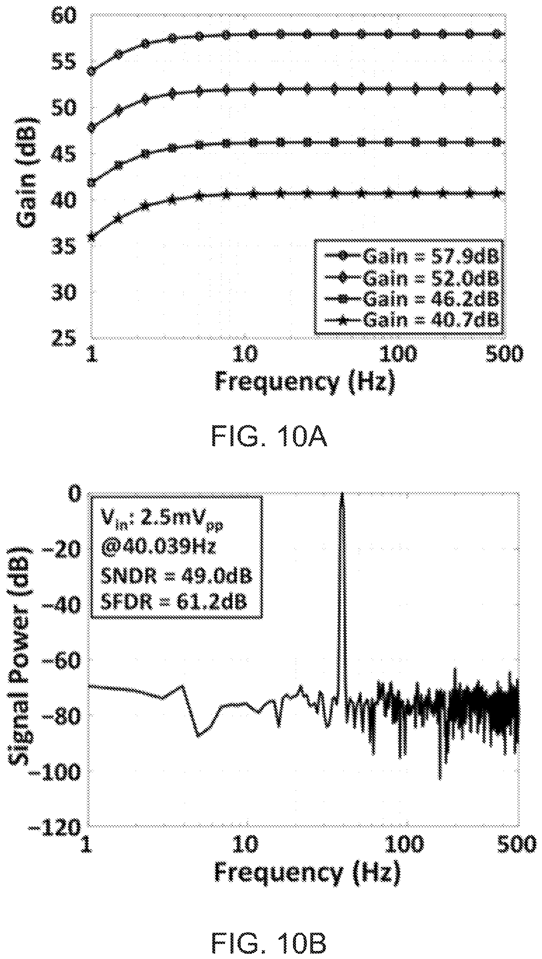

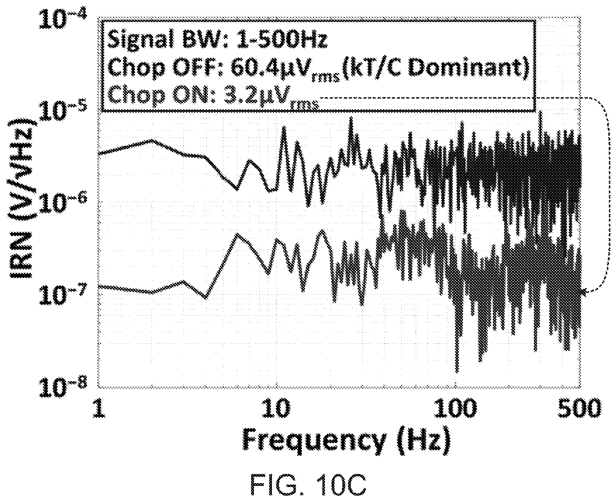

FIGs. 10A-10C show different graphs of measured AFE performance and NeuralTree classification output on two different types of patients, namely epilepsy and Parkinson's disease (PD) patients;

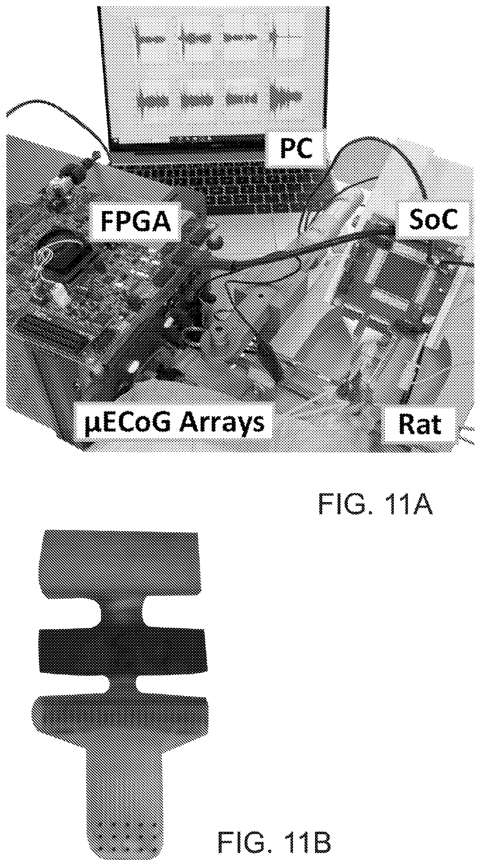

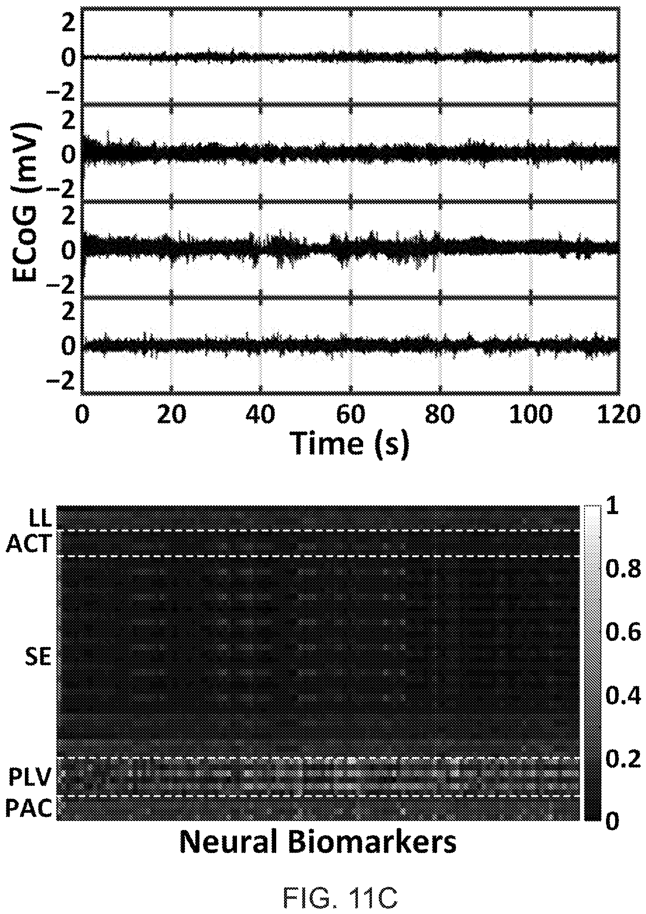

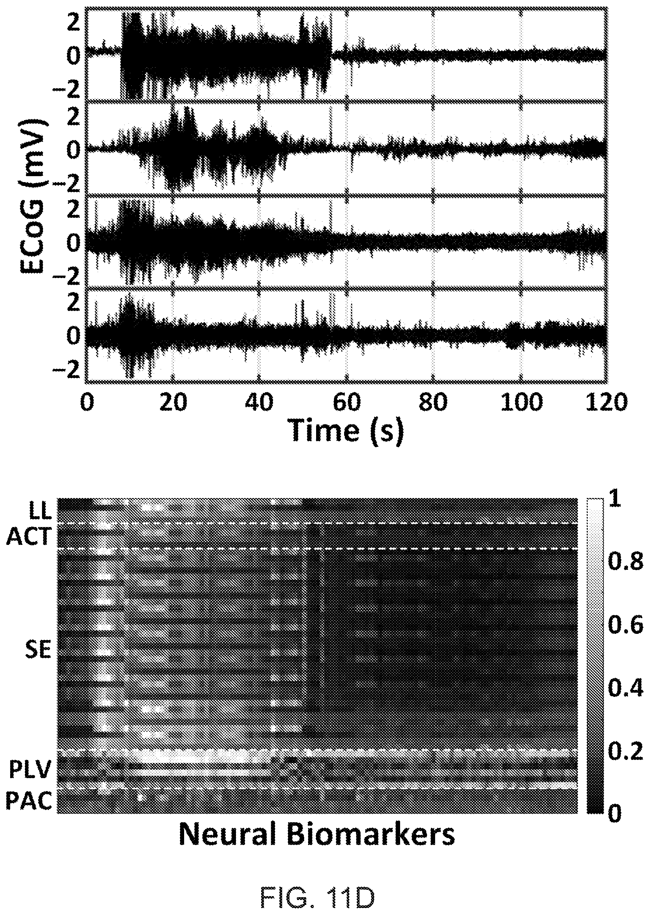

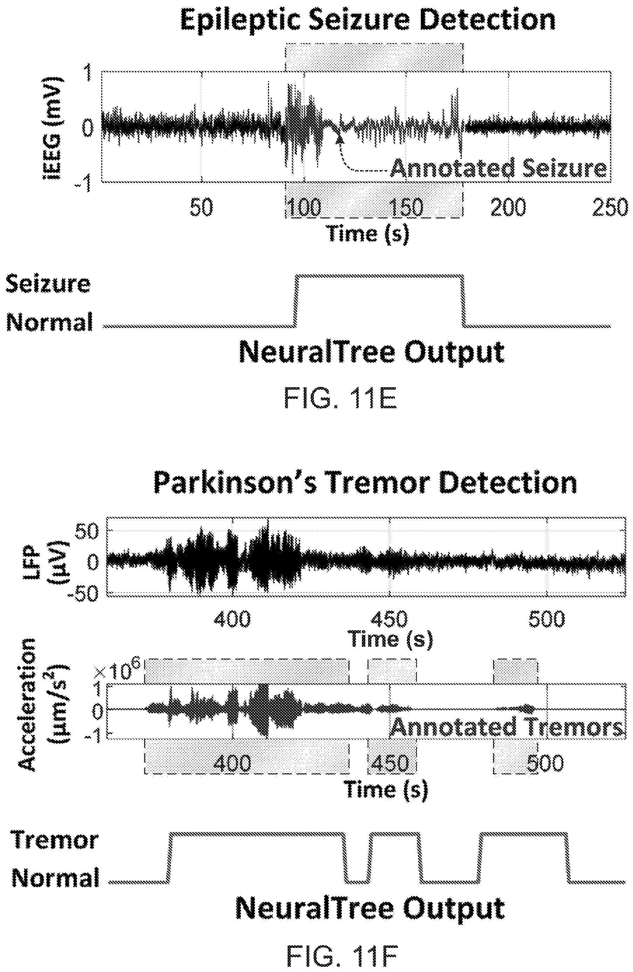

FIG. 11 contains information for SoC validation on a rat model of epilepsy and human datasets, wherein FIG. 11(a) is an experimental setup for *in vivo* testing, FIG. 11(b) a 15-channel soft *µ*ECoG array with a flexible cable, FIG. 11(c) ECoG recordings and neural biomarker extraction in a normal state, FIG. 11(d) ECoG recordings and neural biomarker extraction in a seizure state, FIG 11(e) epileptic seizure detection from iEEG, and FIG. 11(f) Parkinsonian tremor detection from LFPs;

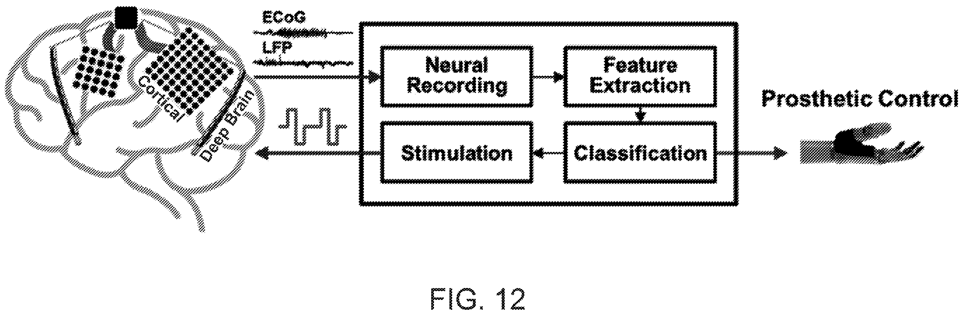

FIG. 12 shows a simplified overview of a closed-loop neuromodulation system, including a prosthetic control device, according to still another aspect of the present invention;

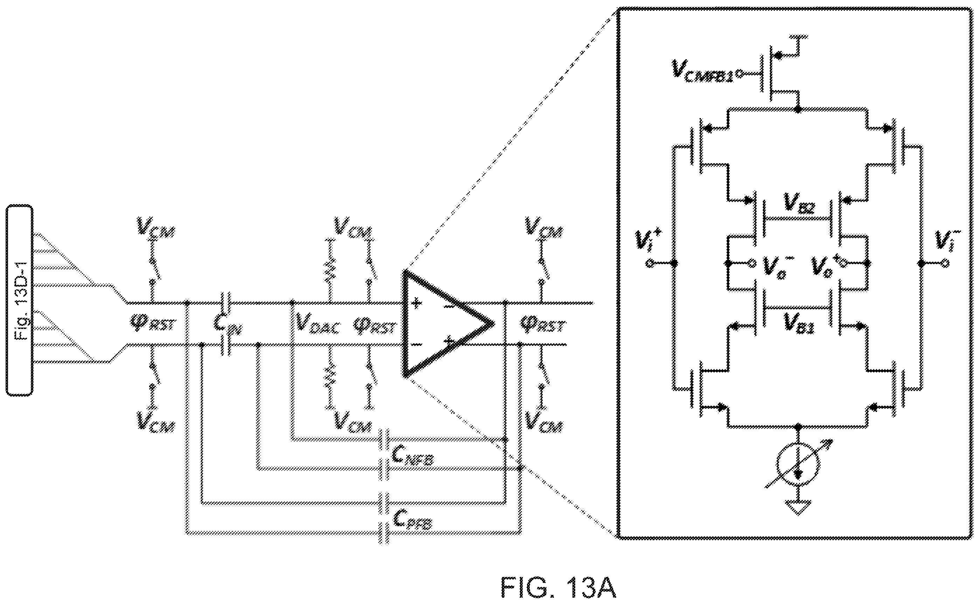

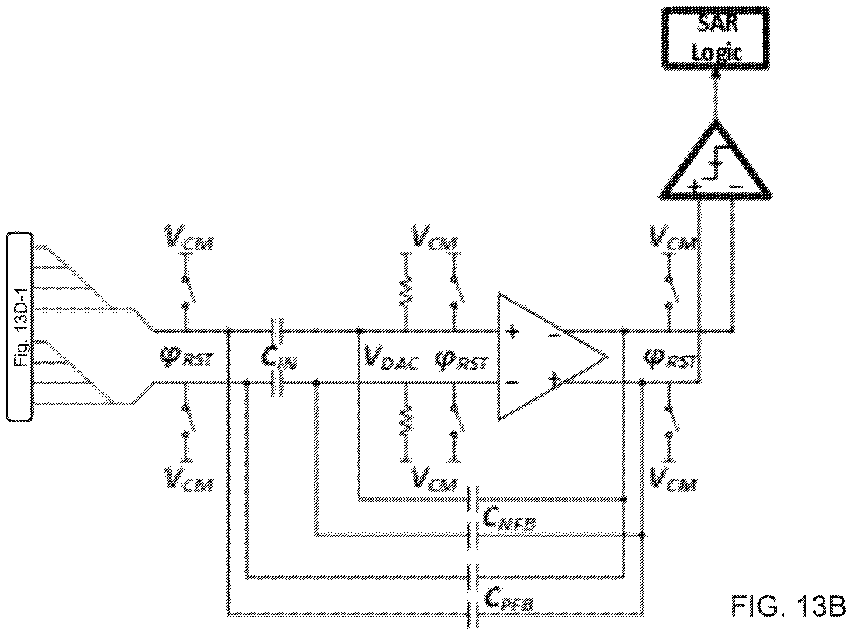

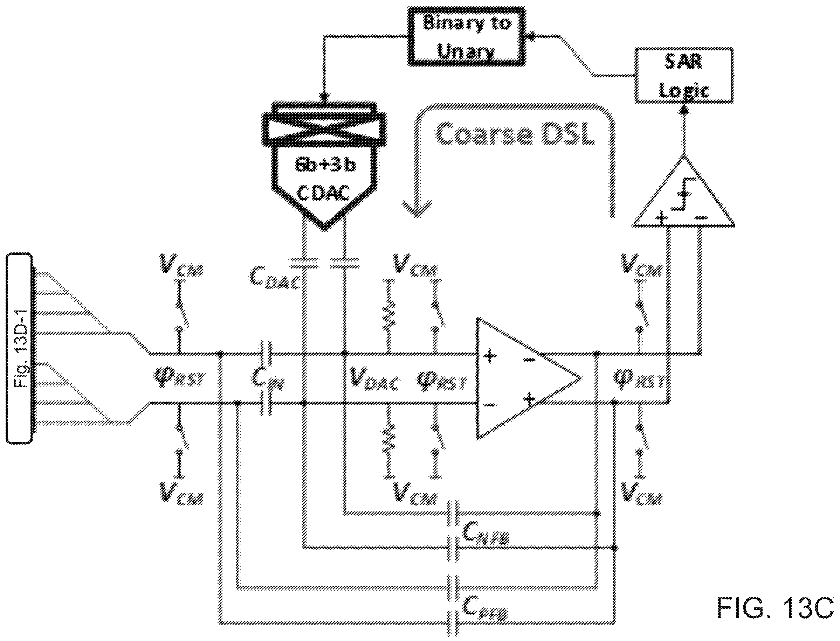

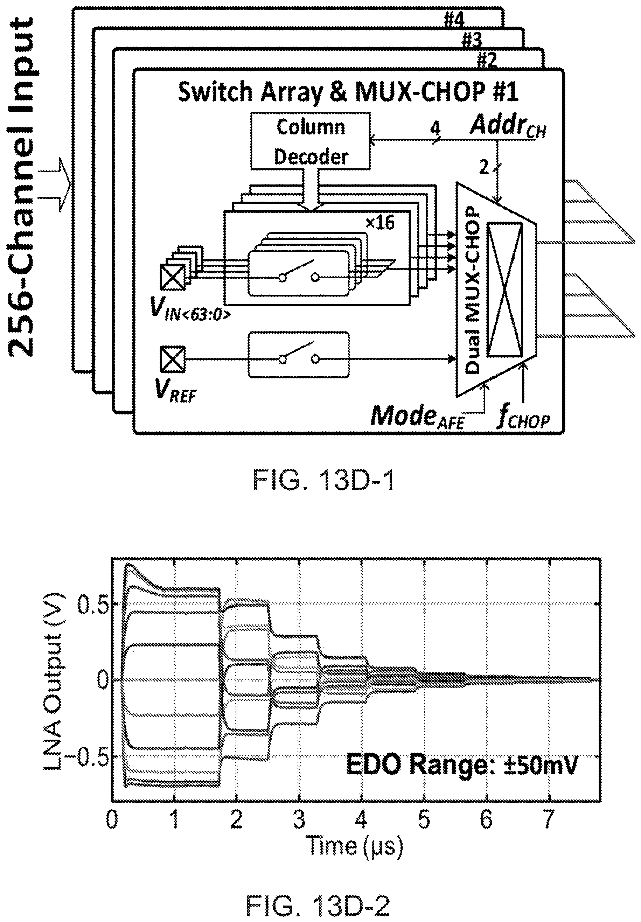

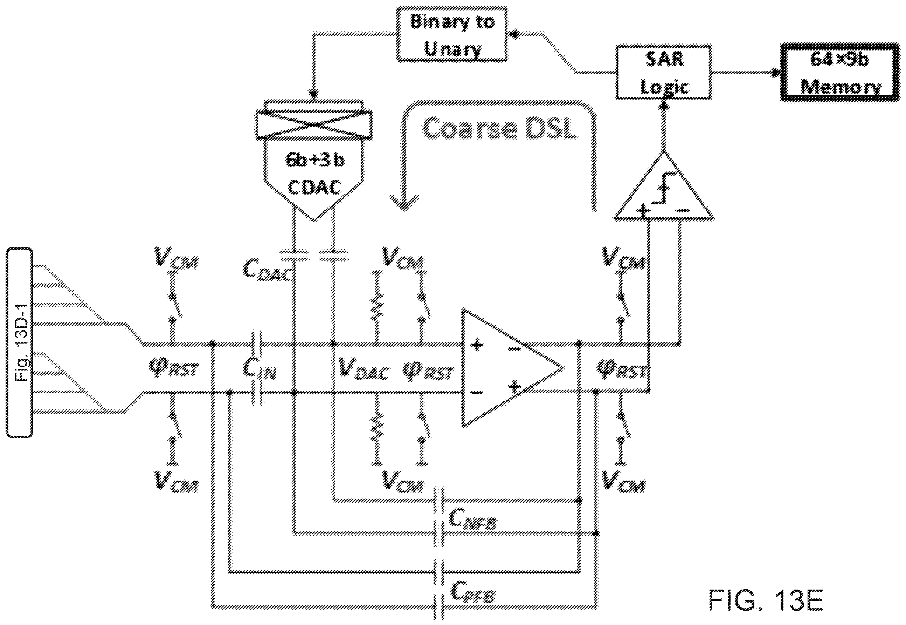

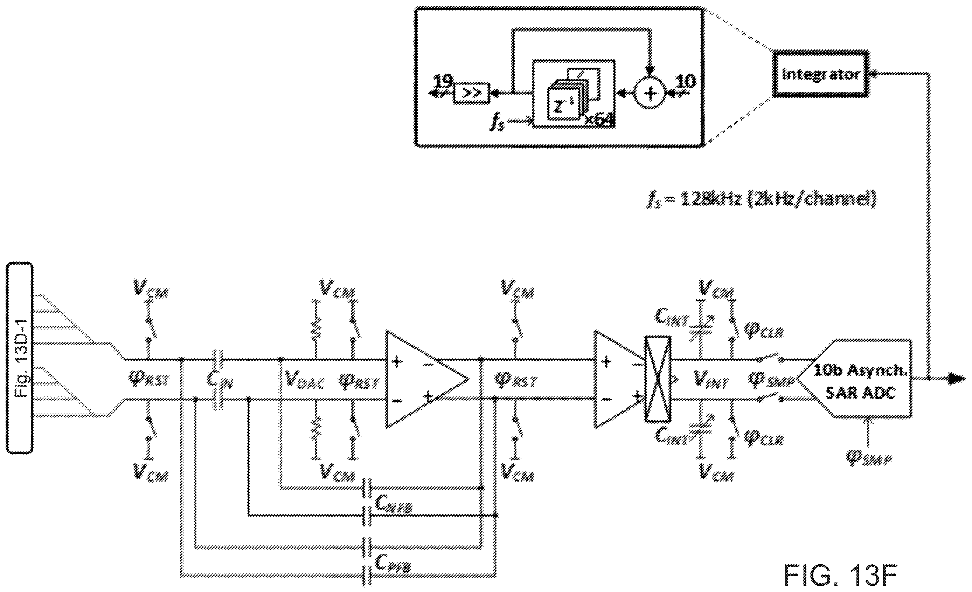

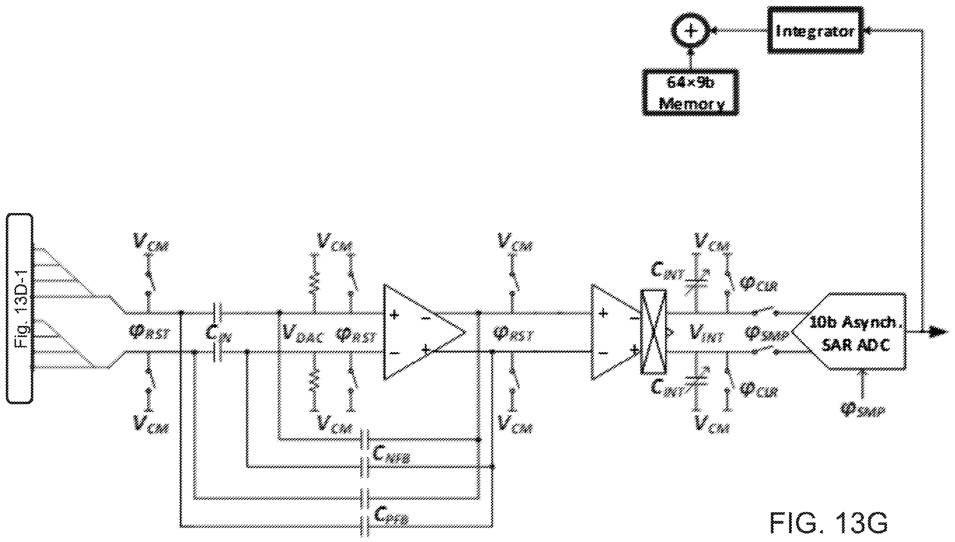

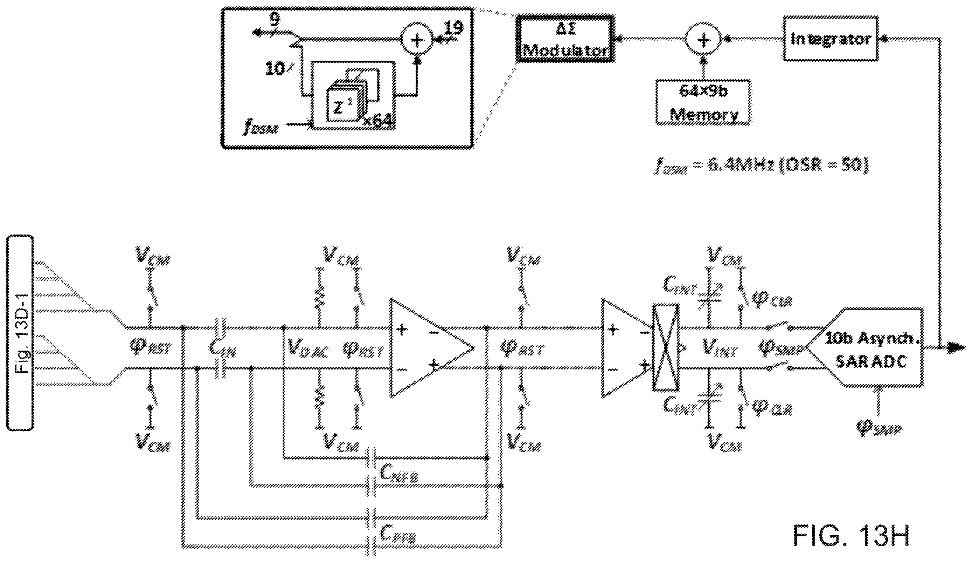

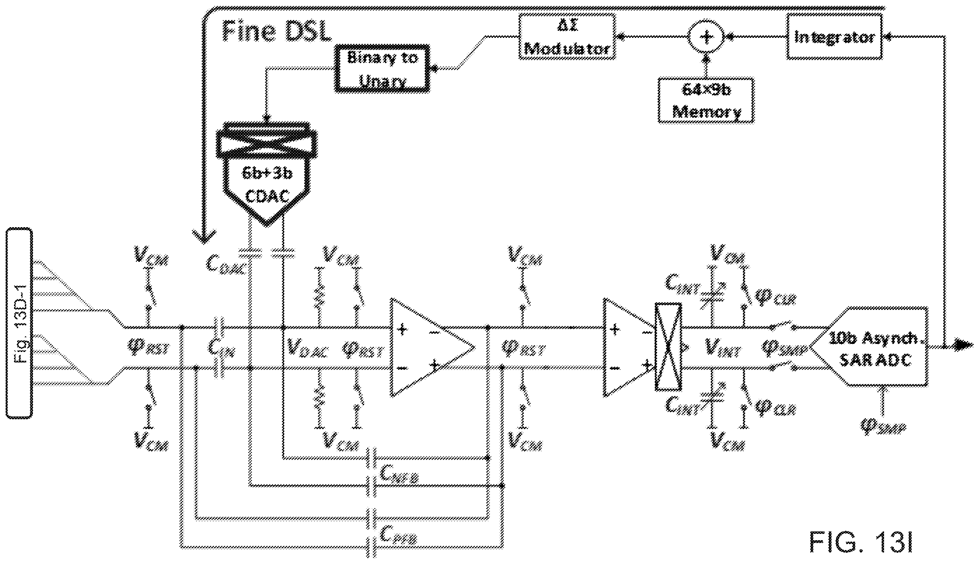

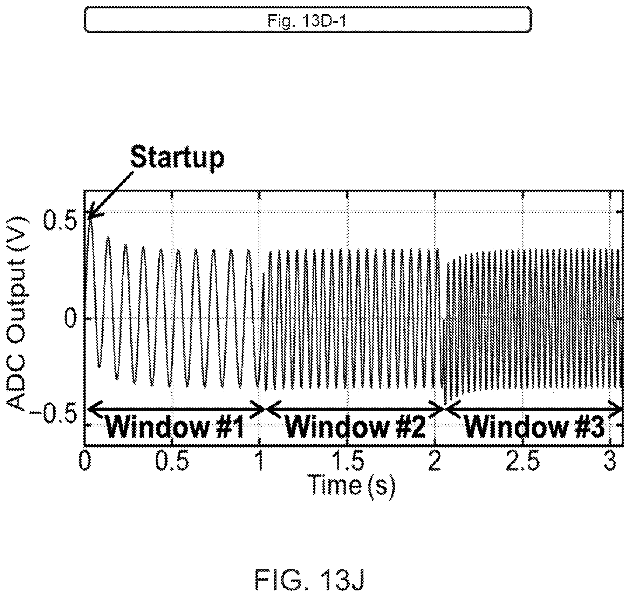

FIG. 13A to 13J illustrate different steps when performing the two-step fast-settling DC servo loop (DSL) method, performed by the analog front-end device (AFE), FIG. 13A illustrating the low-gain high-bandwidth low-noise amplifier (LNA), FIG. 13B illustrating the dynamic comparator and successive approximation register (SAR) logic, FIG. 13C illustrating 9b unary-coded capacitive digital-to-analog converter (CDAC), FIG. 13D illustrating with a graph the rapid 9b EDO cancellation (7.8µs per channel), FIG. 13E illustrating that the EDO is stored in the memory (9b per channel), FIGs. 13A to 13E illustrating the first coarse EDO, next, FIG. 13F the exemplary 64-channel TDM digital integrator of the fine EDO step, FIG. 13G showing that the 19b integrator output and the 9b EDO (MSBs) are added, FIG. 13H illustrating the 64-channel TDM delta-sigma (ΔΣ) modulator, FIG. 13I illustrating the ΔΣ feedback CDAC (oversampling rate per channel = 50), FIG. 13J illustrating with a graph of the fine cancellation of residual EDOs over three successive windows.

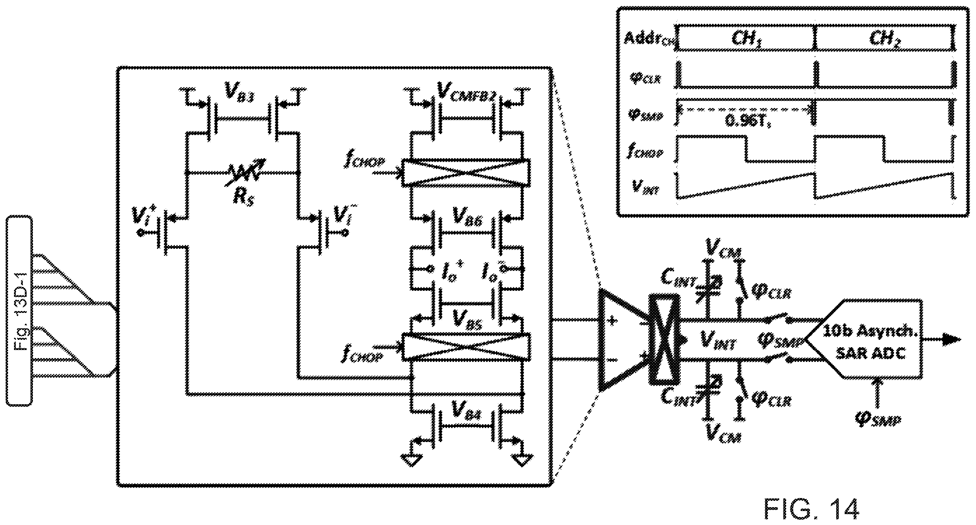

FIG. 14 shows aspects of the analog front-end device (AFE) for Gₘ-C integration for charge sampling, providing a relaxed settling requirement and anti-aliasing sinc filter, and a gain programmability with tunable R_{S} and C_{INT};

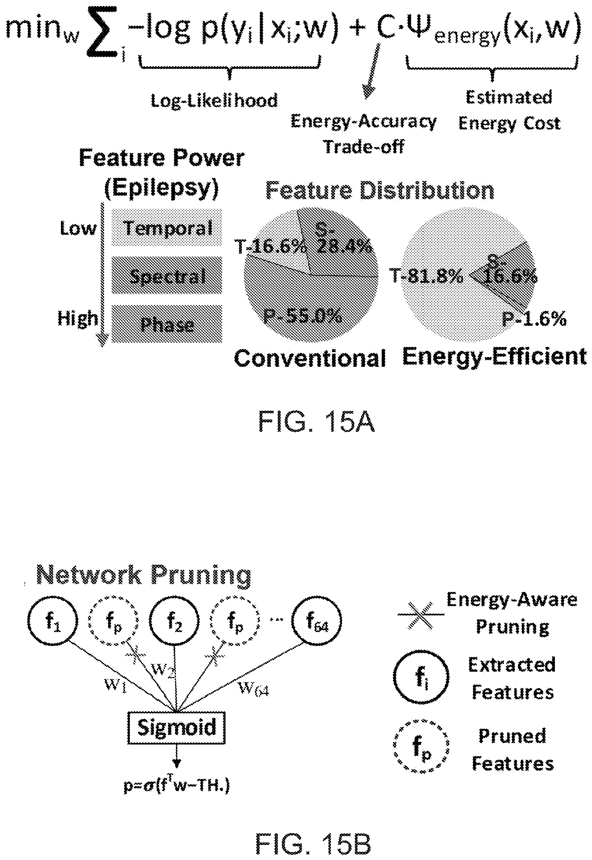

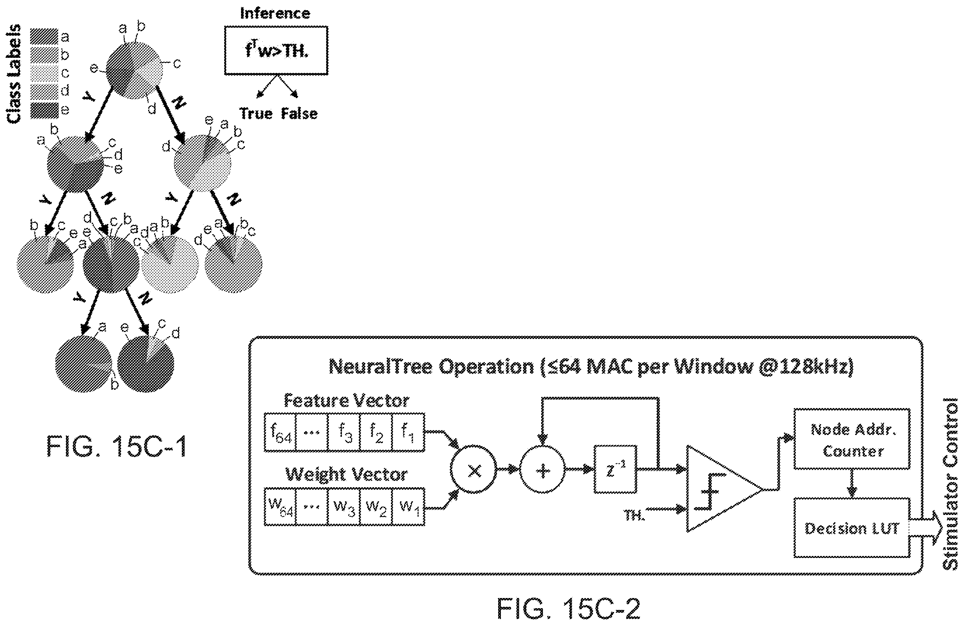

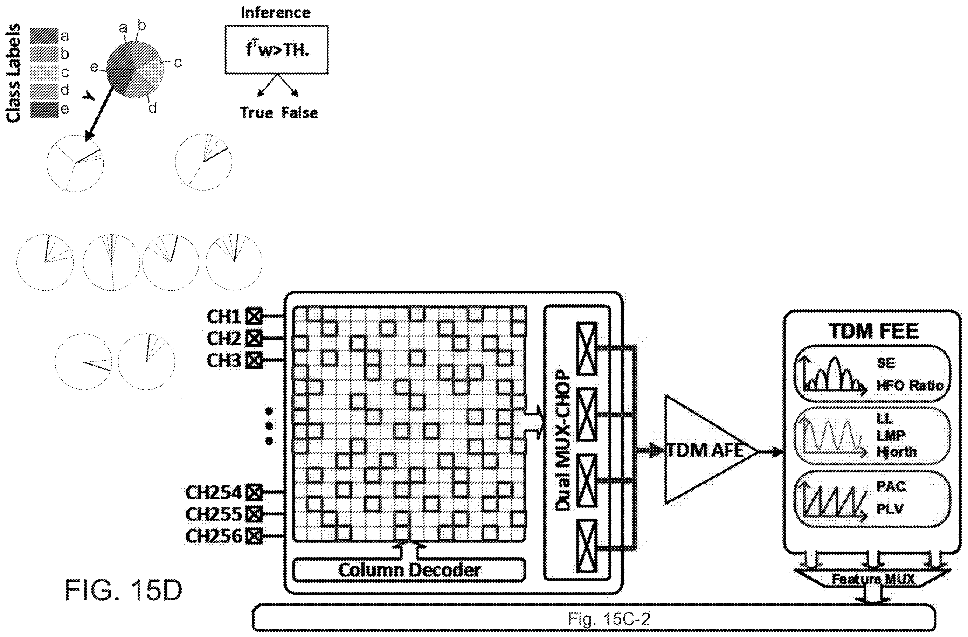

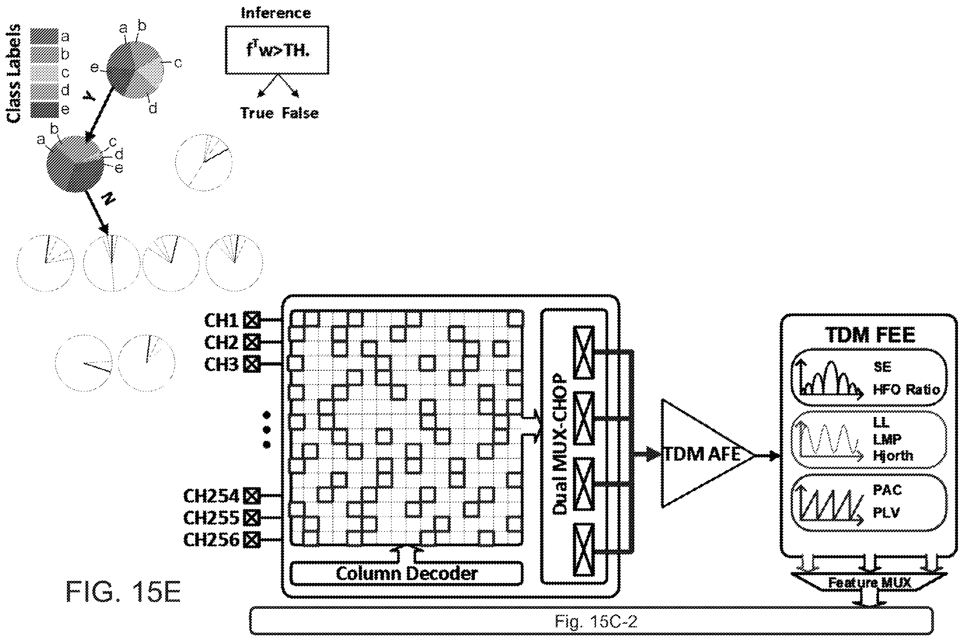

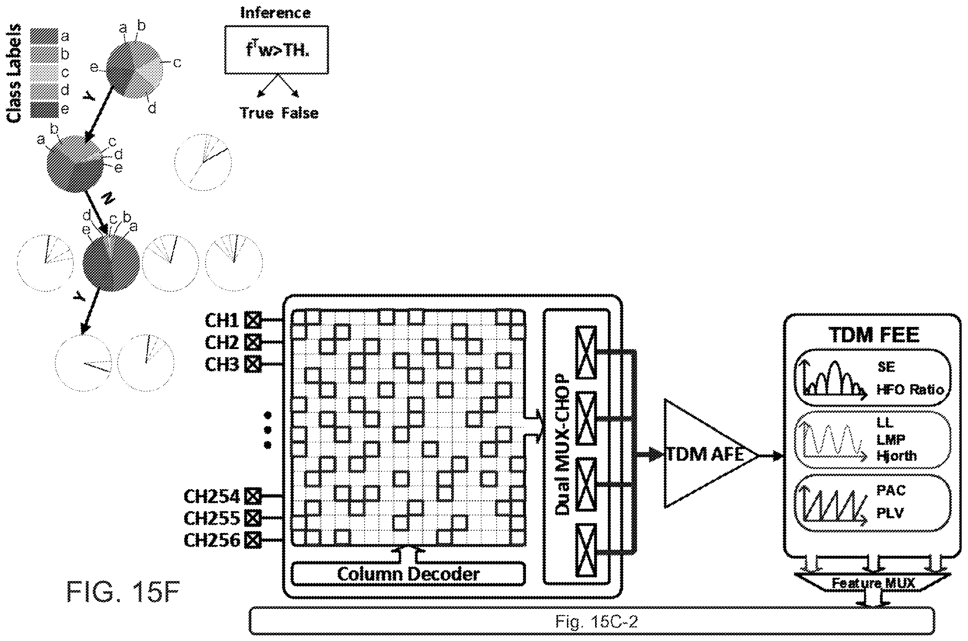

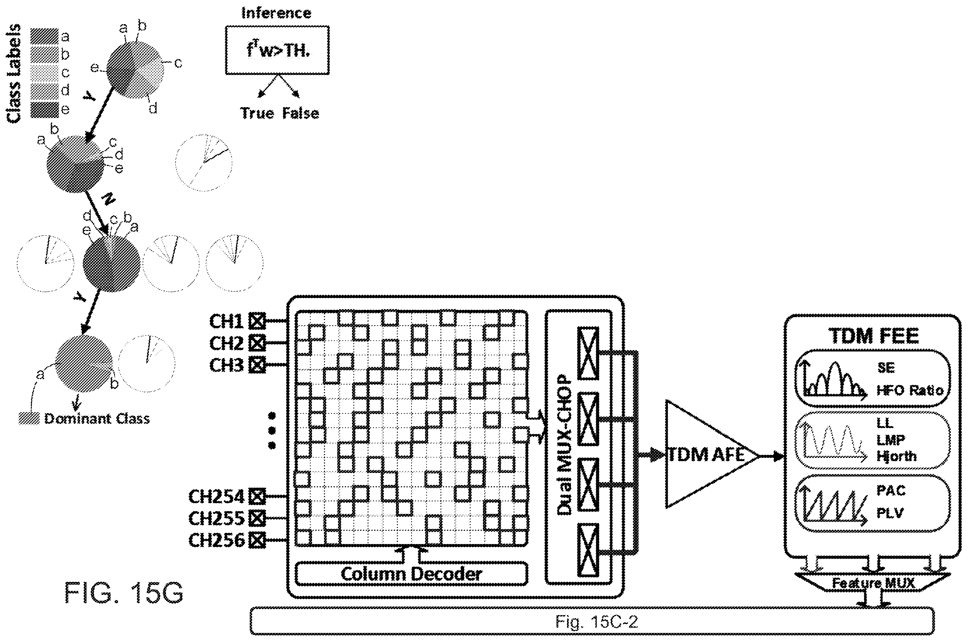

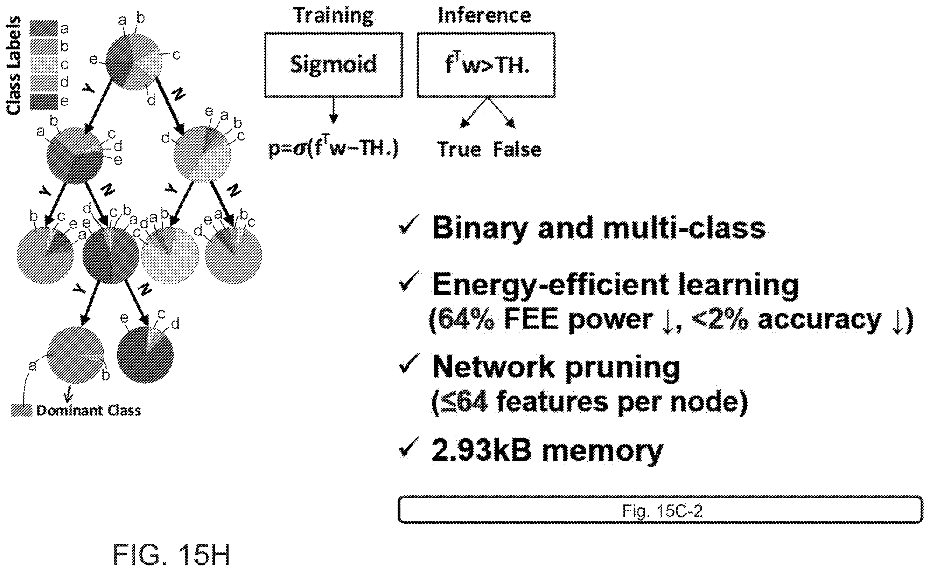

FIGs. 15A to 15H show an exemplary illustration of the different steps performed by the low-power tree-structured hierarchical neural network (NeuralTree) classifier, and aspects of a method of training and configuring the tree-structured hierarchical neural network, according to yet another aspect of the present invention, with FIG. 15A showing the equation for the energy-efficient regularization, to change the features distribution to prioritize energy-efficient features for the neural network classifier, FIG. 15B shows the application of tree pruning combined with energy-efficient regularization by removing power-demanding features from nodes of the tree-structured hierarchical neural network, FIGs. 15C to 15H showing the low-complexity hardware architecture of the NeuralTree classifier as well as the dynamic selection of 64 input channels out of 256 electrodes for successive node processing along the decision path of the pruned NeuralTree;

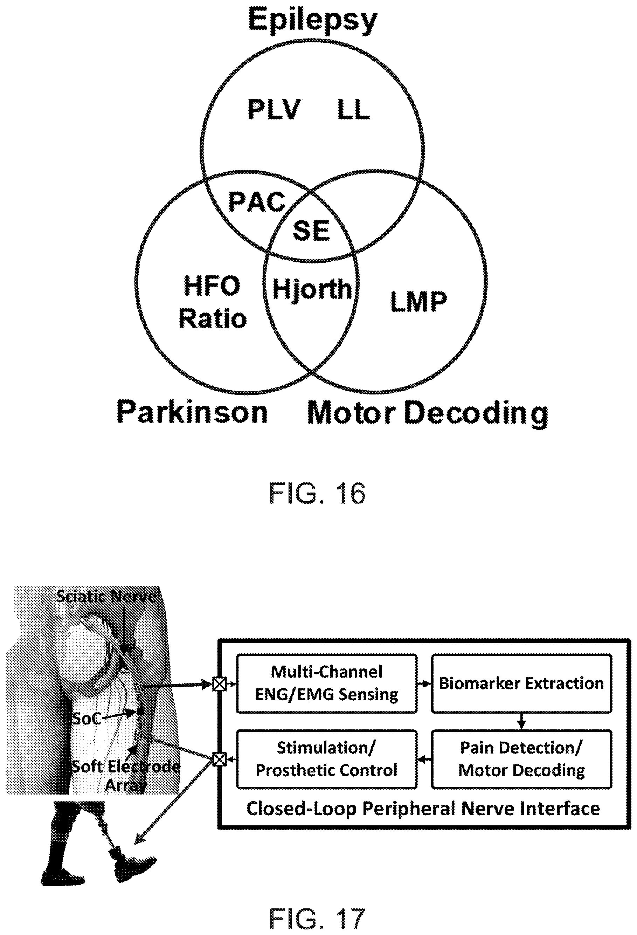

FIG. 16 shows a simplified graphical representation of the different features that can be used for different types of applications, for example the applications for Parkinson's disease, epilepsy, and motor decoding, with some of the features overlapping for different applications, while other are specific to an application;

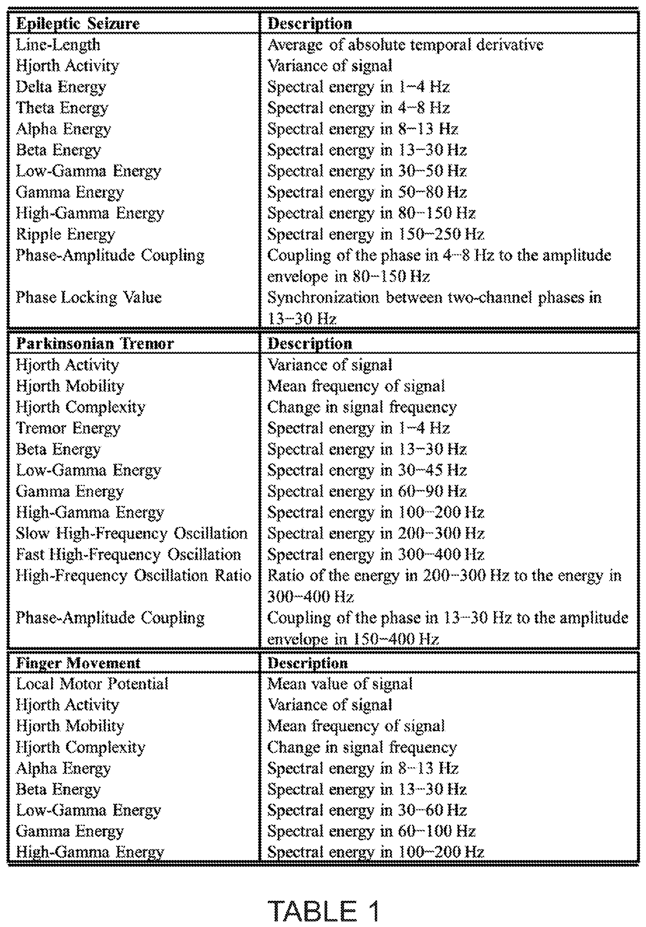

FIG. 17 illustrates an application in an extension to Peripheral Nervous System (PNS) and Spinal Cord application domains; and

TABLE 1 contains task-specific neural biomarkers integrated on the SoC.

Herein, identical reference numerals are used, where possible, to designate identical elements that are common to the figures. Also, the images in the drawings are simplified for illustration purposes and may not be depicted to scale.

### DETAILED DESCRIPTION OF THE SEVERAL EMBODIMENTS

According to at least some aspects of the present invention, a system and a device for neurostimulation is herewith provided, addressing several limitations and problems of the state of the art. For example, a neuromodulation device is provided, preferably a system-on-chip (SoC), that preferably includes (1) a multichannel area-efficient dynamically addressable analog front-end (AFE), exemplarily having 256 channels, (2) information-rich multi-symptom biomarkers, (3) a low-power tree-structured hierarchical neural network (NeuralTree) classifier, and (4) a multichannel high-voltage (HV) compliant neurostimulator, exemplarily having 16 channels.

FIG. 1 depicts a schematic of an exemplary view of the SoC architecture **100.** Four 64-channel chopper-stabilized time-division-multiplexed (CS-TDM) analog front-end (AFE) modules **101** are shown that can record 256-channel neural signals from a plurality of electrodes, for example from a cortical grid **102,** to train the classifier via a 16×16 switch matrix **103** and dual multiplexing choppers **104** (MUX-CHOP). The AFE inputs are reconfigured in inference mode such that any subset of 64 informative channels can be dynamically multiplexed to the main AFE module (#1) by a column decoder **105** and dual choppers **104** on a window-by-window basis. The digitized neural signals are passed to a time-division-multiplexed (TDM) finite impulse response (FIR) filter **106** and a TDM feature extraction engine **107** (FEE) that generates patient-specific neural biomarkers. The end-to-end TDM implementation enables efficient multi-channel processing without the need for demultiplexing. The low-power tree-structured hierarchical neural network (NeuralTree) supports both binary and multi-class classification. Upon detection of disease symptoms or movement type, the stimulator **108** is triggered to close the therapeutic loop.

As only subsets of channels contain relevant information to predict a specific disease state, for example but not limited to Parkinson's disease or epilepsy, high-density training followed by channel-selective inference can drastically reduce hardware complexity while retaining classification accuracy.

FIG. 2 contains a graph showing a relative importance of 128 iEEG channels from an epileptic patient and evaluated with 5-fold cross-validation.

As the sensor count increases, the area constraint on the AFE becomes more stringent and the complexity of the back-end signal processing also grows significantly. We tackle these challenges with an area-efficient TDM AFE with a channel-selective inference scheme. Noting that only subsets of input electrodes capture disease-relevant neural activity, the channel-selective approach can greatly reduce the hardware overhead during inference. To validate this concept, we trained a classifier on 128-channel intracranial electroencephalography (iEEG) recorded from an epileptic patient to assess the discriminative power of each channel. The NeuralTree classifier (detailed in Section IV-C) was trained using two common types of seizure biomarkers [line-length (LL) and multi-band spectral energy (SE)] extracted from the 128 channels. The importance of each channel was then assessed based on the number of features extracted during inference using 5-fold cross-validation. The nonuniform channel importance in FIG. 2 implies that high-density training followed by channel- selective inference can save the inference cost significantly while maintaining the classification accuracy.

### Multi-Symptom Feature Extraction

To enhance the versatility of the system-on-chip (SoC) for a broad range of neural classification tasks, the feature extraction engine (FEE) integrates multi-symptom neural biomarkers, as summarized in Table I. Without careful design considerations, integrating such a broad range of biomarkers can be hardware intensive. This Section describes hardware-friendly feature approximation algorithms and circuit techniques that enable low-complexity, yet accurate feature extraction in the proposed SoC.

1) Temporal Features: Line-length (LL) increases in the presence of high-amplitude or high-frequency neural oscillations and has been among powerful biomarkers of epileptic seizures. LL is defined as follows: $LL = \frac{1}{N} {\sum }_{t = 1}^{N} \left|{x}_{t}-{x}_{t - 1}\right|$
where N is the number of samples in a feature extraction window. FIG. 3(a) presents the hardware implementation of the proposed time-division multiplexed (TDM) temporal feature extractor. For LL extraction, the absolute differences between successive samples are accumulated with two adders and one absolute value calculator.

The Hjorth statistical parameters are highly correlated with tremor in Parkinson's disease (PD) and used in brain-machine interfaces (BMIs) for finger movement and gait decoding. The Hjorth activity (ACT), mobility (MOB), and complexity (COM) measure the variance, mean frequency, and frequency change of a signal, respectively, as defined in the following: $ACT = var \left(x\right) = \frac{1}{N} {\sum }_{t = 1}^{N} {\left({x}_{t}-μ\right)}^{2}$ $MOB = \sqrt{\frac{var \left(Δx\right)}{var \left(x\right)}}$ $COM = \sqrt{\frac{var \left(x\right) ⋅ var \left({Δ}^{2}x\right)}{{var}^{2} \left(Δx\right)}}$
where *µ*, Δ*x,* and Δ²*x* are the mean, first, and second derivatives of the signal *x*, respectively.

The three Hjorth parameters are difficult to efficiently compute in their original form, due to the intensive multiplication and square-root operations. Goncharova and Barlow introduced a similar set of parameters, namely, mean amplitude, mean frequency, and spectral purity index (SPI), in which the square and square-root operators are replaced by simple absolute value approximations. These new parameters are less intensive to compute while preserving a close relation to the measures of EEG amplitude and frequency. We adopt this approach to approximate the Hjorth features as in (5)-(7), with a modification to the SPI parameter by taking its reciprocal, since it is better correlated with the original Hjorth complexity parameter $ACT \approx \frac{1}{N} {\sum }_{t = 1}^{N} \left|{x}_{t}\right|$ $MOB \approx \frac{{\sum }_{t = 1}^{N} \left|Δ{x}_{t}\right|}{{\sum }_{t = 1}^{N} \left|{x}_{t}\right|}$ $COM \approx \frac{\left({\sum }_{t = 1}^{N} \left|{x}_{t}\right|\right) ⋅ \left({\sum }_{t = 1}^{N} \left|{Δ}^{2}{x}_{t}\right|\right)}{{\left({\sum }_{t = 1}^{N} \left|Δ{x}_{t}\right|\right)}^{2}}$

To calculate the approximated Hjorth features, the absolute values of the input and its first and second derivatives are accumulated selectively, as shown in FIG. 3(a). For MOB and COM extraction, the subsequent multipliers and ratio calculator further process the accumulated derivatives to compute features in fractional form. The ratio calculator employs a reciprocal-multiply approach with bit shifting instead of a complex divider, as depicted in FIG. 3(a).

Local motor potential (LMP) has been used as a low-complexity yet effective marker for motor intention decoding in BMIs. The LMP feature quantifies the mean value of a signal as defined in the following: $LMP = \frac{1}{N} {\sum }_{t = 1}^{N} {x}_{t}$

The accumulation function can be performed by reusing the ACT extractor and bypassing the absolute value calculator, as shown in FIG. 3(a).

2) Spectral Features: Spectral energy (SE) in multiple frequency bands of neural oscillations has been a commonly used biomarker in epilepsy, and BMIs. As a measure of signal power integrated over time, the SE can be defined in the discrete-time domain as follows: $SE = \frac{1}{N} {\sum }_{t = 1}^{N} {x}_{BAND , t}^{2}$
where *x_{BAND,t}* indicates the bandpass-filtered neural signal.

A common approximation method to avoid the square operation is to take the absolute output of the bandpass filter. The 16-channel EEG processor for example demultiplexed the output of the TDM finite impulse response (FIR) filter to 112 signal paths (16 channels × 7 bands) to calculate 112 SE features in parallel. This approach requires an equal number of multi-bit adders and absolute value calculators with a significant area overhead. To save chip area, the TDM spectral feature extractor in FIG. 3(a) directly receives the BPF output as the input without demultiplexing and extracts up to 64 SE features using a single adder. The area efficiency is further improved by reusing the hardware already implemented for ACT and LMP extraction.

High-frequency (>200 Hz) oscillations (HFOs) are prominent features in PD and epilepsy (>80 Hz). For instance, it has been reported that the energy ratio between the slow (HFO1, 200-300 Hz) and fast HFO (HFO2, 300-400 Hz) as an indicator of rest tremor in PD: ${HFO}_{R} = \frac{{\sum }_{t = 1}^{N} {x}_{{HFO}_{1} , t}^{2}}{{\sum }_{t = 1}^{N} {x}_{{HFO}_{2} , t}^{2}}$

The SE extractor is reused to calculate the slow and fast HFOs, while the ratio between the two is computed using the ratio calculator shared with the Hjorth feature extractor.

3) Phase Features: Different brain regions communicate with each other through neuronal oscillations. Abnormal cross-regional synchronization of neural oscillations can indicate disease-related pathological states in neurological and psychiatric disorders. In epilepsy, spatial and temporal changes in cross-channel phase synchronization, quantified by phase locking value (PLV), play as a key indicator of seizure state. Phase-amplitude coupling (PAC) is another mechanism for within- and cross-regional brain communication. PAC quantifies the degree to which the low-frequency neural oscillatory phase modulates the amplitude of HFOs. Excessive PAC has been observed in disorders such as epilepsy, PD, and depression.

Measuring PLV and PAC requires Hilbert transform (HT) to obtain analytic signals followed by several complex computations, such as extraction of instantaneous phase and amplitude, trigonometric functions, and magnitude computation, as shown in the following: $PLV = \frac{1}{N} \sqrt{{\left({\sum }_{t = 1}^{N} sin Δ {θ}_{t}\right)}^{2} + {\left({\sum }_{t = 1}^{N} cos Δ {θ}_{t}\right)}^{2}}$ $PAC = \frac{1}{N} \sqrt{{\left({\sum }_{t = 1}^{N} {A}_{t} {sinθ}_{t}\right)}^{2} + {\left({\sum }_{t = 1}^{N} {A}_{t} {cosθ}_{t}\right)}^{2}}$
where Δ*θₜ* in (11) is the cross-channel phase difference, and *θₜ* and *Aₜ* in (12) are the modulating phase and modulated amplitude envelope, respectively.

The SoCs from prior art for example employed multiple COordinate Rotation DIgital Computer (CORDIC) processors to compute these non-linear functions, consuming an excessive amount of power (>200 µW). Alternatively, FIG. 3(b) depicts the proposed TDM phase feature extractor. With band-specific analytic signals (Re and Im) as inputs, the instantaneous phase is approximated using a linear arctangent approximation (LAA) algorithm followed by lookup table (LUT)-based error correction. The *l*_{∞}-norm is used to approximate the amplitude envelope of HFOs in PAC, as well as the magnitude computations in (11) and (12). The TDM phase feature extractor can compute up to 32 PLV/PAC features on demand in a compact area of 0.033 mm².

To evaluate the accuracy of the proposed feature approximation algorithms, we analyzed the Pearson correlation coefficient between the ideal and approximated features in MATLAB. The phase and eight-band SE features were extracted from an epilepsy iEEG dataset, while a PD local field potential (LFP) dataset was used to compute the HFO ratio and Hjorth features.

Thanks to feature approximations and hardware sharing, the proposed multi-symptom FEE occupies a small silicon area of 0.12 mm², even with the complex features integrated. Aggressive hardware sharing among different feature calculators is possible thanks to the on-demand TDM scheme, where only selected features are consecutively extracted. With a 128-kHz clock, the FEE can generate any combination of up to 64 neural biomarkers in each programmable feature extraction window (0.25-2 s). Any unused hardware units are selectively clock- and data-gated to reduce dynamic power dissipation.

FIG. 4 schematically illustrates the multichannel CS-TDM AFE **101** having an exemplary number of 256 channels. A major challenge to performing dynamic channel selection for brain activity inference is electrode DC offsets (EDOs) that fluctuate between input channels in successive feature extraction windows, in contrast to conventional TDM designs. To address this issue, a two-step fast-settling mixed-signal DC servo loop (DSL) is proposed, including a coarse DSL **200** and a fine DSL **201.** In the coarse EDO cancellation step, 1) the EDO in each of the selected input channels is amplified by a low-noise amplifier (LNA), 2) the polarity of the EDO is detected by a comparator, 3) the one bit (1b) comparator output is stored in a successive approximation register (SAR) logic, and 4) a 9b capacitive digital-to-analog converter (CDAC) is configured to coarsely subtract the EDO from the input signal. This binary search process is repeated 9 times for each input channel rapidly to search for 9 bit digital signal representations (9b) of 64-channel EDOs (±50mV) and store them into a local memory (9b/channel) within the first sampling period of each window.

Next, neural recording begins with the fine DSL **201** enabled. The digital integrator extracts undesired low-frequency signal components including residual EDOs. The integrator output and the pre-stored 9b EDO are added, delta-sigma (ΔΣ) modulated, and fed back to the input through the 9b CDAC to remove residual EDOs. The mixed-signal DSL facilitates multi-channel EDO cancellation and is more area-efficient than analog S/H-based DSLs.

A closed-loop LNA with a current-reuse amplifier is implemented for improved noise efficiency. A Gₘ-C integrator provides gain programmability and anti-aliasing. The intermediate nodes are periodically reset to reduce crosstalk between adjacent channels. The kT/C noise, which is sampled as a result of this reset operation, is up-modulated by a chopper (the rectangular cross box on the lower side of FIG. 5C) and filtered out by the anti-aliasing Gm-C integrator so that it does not degrade the overall noise performance of the AFE . In an exemplary and non-limiting embodiment, the AFE having an exemplary 256 channels can occupy a chip surface area of 1.1mm² with 0.004mm²/channel and consumes 387µW (1.51µW/channel) in training mode. The AFE power consumption is reduced to 182µW during an exemplary 64-channel inference by disabling the LNAs and DSLs in three auxiliary AFEs. This scalable and modular AFE enables 8× higher channel count over SoCs of the state of the art, and improved power and area per channel compared to other high-density AFEs of the state of the art.

FIG. 6 shows different exemplary schematics of the time-division-multiplexed (TDM) finite impulse response (FIR) filter device receiving input signals from the AFE device, the TDM feature extraction engine (FEE) having three different feature extractors including a phase-synchrony feature extractor, a spectral feature extractor, and a temporal feature extractor, and also showing a more detailed view in the lower section of the phase-synchrony feature extractor of the FEE including a linear arctangent approximation device (LAA).

To enhance the versatility of the herein presented device or system, for example an SoC, the FEE provides rich patient- and disease-specific feature extraction, including but not limited to (1) spectral (multi-band spectral energy, high-frequency oscillation ratio), (2) phase synchrony (phase locking value, PLV, and phase-amplitude coupling, PAC), and (3) temporal features (line-length, local motor potential, LMP, and Hjorth statistical parameters). Depending on feature types required by the trained classifier, the exemplary 64-channel neural signals are sequentially processed by the FEE. As an exemplary embodiment for the TDM FIR, a programmable 32-tap TDM FIR reuses a single set of arithmetic units (for example multipliers and adders) for multi-channel (≤ 64) bandpass filtering of the digitized AFE output. The FIR can be reconfigured as a 31-tap Hilbert transformer to obtain analytic signals for instantaneous phase and amplitude envelope extraction of the bandpass-filtered neural signals. For Hilbert transformation, the AFE output is first bandpass-filtered by the bandpass FIR filter, which can include 32-tap first-in first-out (FIFO) registers, multipliers, and adders. The bandpass FIR filter output can then be sent to 31-tap FIFO registers. Thereafter, the Hilbert transformation is performed by the same arithmetic hardware with a different set of FIR coefficients, which is retrieved from the FIR coefficient memory. It has been shown that the cross-region phase synchrony has strong preclinical evidence for early diagnosis and closed-loop intervention in neurological disorders. Accurate low-power computation of instantaneous phase is essential for realizing such features on chip.

FIG. 6 also depicts the phase feature extraction hardware with a linear arctangent approximation (LAA) algorithm. In LAA, the input range is reduced to the first quadrant in the complex plane, and the instantaneous phase is coarsely approximated to a fraction of the two inputs (Re, Im) of the complex plane, using reciprocal multiplication. Accurate 10 bit (10b) digital instantaneous phase outputs of the LAA are generated following look-up table (LUT)-assisted error correction and range reconstruction, or other type of error correction, for example one relying on prestored correction data. In the exemplary embodiment, the TDM phase feature extractor can occupy a compact surface area of the chip of 0.033 mm² and consumes 28.7 µW for simultaneous extraction of 32 PAC/PLVs while maintaining accuracy (>95% correlation with ideal features). Compared to a conventional CORDIC-based phase feature extractor, a seven-fold improvement in power consumption was achieved, as shown in the middle section comparing power consumption of FIG. 5.

FIG. 7 shows an exemplary schematical representation of the low-power tree-structured hierarchical neural network (NeuralTree) classifier, generating control signals for stimulating a brain of a patient or user with the plurality of implanted electrodes, for example the cortical grid, the NeuralTree classifier preferably trained with mini-batch gradient descent under a stochastic routing scheme, as shown in Zhu et al., "ResOT: Resource-Efficient Oblique Trees for Neural Signal Classification," IEEE TBioCAS, vol. 14, no. 4, pp. 692-704, June, year 2020. With the aspects of FIG. 7, details of the hardware implementation of NeuralTree are shown, and altogether these aspects result in superior results in terms of low power, small area, and small memory usage for this neural classifier compared to state-of-the-art.

Considering that most samples are routed with high certainty, the inference can be simplified to conditional computations, avoiding complex sigmoid functions. Each internal node is represented by a 2-layer sparsely connected neural network (NN). Through network pruning, the number of features extracted per node is reduced by 87.0% on average (≤64 per node), improving energy efficiency and scalability. In contrast to recent SoCs that use large (e.g., 1024-tree) ensembles, the stand-alone NeuralTree significantly reduces feature count and also surface area for a chip implementation. Furthermore, a single NeuralTree can handle multi-class tasks (e.g., finger movement classification) with marginal memory overhead (i.e., extra bits needed to store class values). This results in a more efficient approach as compared to stacking binary classifiers in a conventional one-vs-all approach. A single multiply-and-accumulate (MAC) and comparator are reused for successive node processing during inference. The classifier employs neural network pruning, weight and threshold quantization, and energy-efficient regularization, as illustrated in FIG. 7, to improve the energy efficiency. For energy-efficient regularization, we introduce a regularization term that incorporates the hardware cost for feature extraction, see FIG. 7, bottom left. This equation for the regularization adds a power-dependent regularization term to the objective function during training, by estimating the power dissipation of the features used by the NeuralTree model. The feature costs are averaged over samples to take into account the frequency of visiting a node during training. Using this technique, exemplary results have shown that the power consumption for the FEE is reduced by 64%, with less than 2% accuracy loss. Due to a compressed model structure as a result of pruning and quantization, the trained parameters take up only 2.93kB of on-chip memory. This is much smaller than previous designs of the state of the art with an on-chip classifier such as support vector machines (SVM).

When configured to control a prosthetic device, the NeuralTree can operate as a multi-class classifier. For example, the NeuralTree can provide control signals to control different types of prosthetic devices, for example to provide for control signals to move different fingers of a prosthetic hand, to stimulate muscles and initiate movements, or provide sensory feedback to the brain in the form of an electrical stimulation controlled by the NeuralTree.

FIG. 8 showing a schematic and exemplary representation of a high-voltage (HV) compliant multi-channel neurostimulator for interconnection to the plurality of electrodes that can be implanted to a brain, using an exemplary number of 16 channels, enabling closed-loop neuromodulation, an exemplary and non-limiting 16-channel stimulator is implemented using a stacked HV compliant (8V) architecture in standard 65nm process. The charge pump (CP) utilizes high-frequency clocking and a multi-phase technique, thus eliminating the need for large flying capacitors. A sink-regulated H-bridge output driver with a single current source is implemented. Due to the inherent matching of this architecture, simple active charge balancing (CB) with offset regulation followed by passive discharging ensures low residual voltage (<±10mV). To further reduce chip area, every four bipolar channels share a single charge pump (CP) and a level shifter and are individually addressable. The stimulator occupies a small area of 0.05mm²/channel, three times better than the stacked architecture of the state of the art, with a charge mismatch of <0.1%.

FIG. 9 shows the chip micrograph. The exemplary 256-channel SoC occupies a silicon area of 3.48mm².

The AFE gain programmability, dynamic performance, and input-referred noise are presented in FIGs. 10A to 10C. Crosstalk between channels was less than -79.8dB intermodule and -72.8dB intra-module.

In Vivo Measurements: The neural recording and biomarker extraction capabilities of the SoC were validated in vivo in the experimental setup shown in FIG. 11(a). The animal experiments were performed with the approval of all experimental and ethical protocols and regulations granted by the Veterinary Office of the Canton of Geneva, Geneva, Switzerland, under License No. GE/33A (33223). All procedures were in accordance with the Regulations of the Animal Welfare Act (SR 455) and the Animal Welfare Ordinance (SR 455.1). We implanted two 15-channel 200-µm-diameter soft µECoG arrays, shown in FIG. 11(b), into the somatosensory cortex of a Lewis rat. The µECoG arrays were fabricated using an e-dura technology with gold thin films. Pentylenetetrazol (20 mg) was injected intraperitoneally into the anesthetized rat to induce seizures. FIG. 11(c) and (d) presents ECoG recordings and neural biomarkers in a normal and seizure state, respectively. Prominent increases in temporal and spectral biomarkers at the seizure onset and strong cross-channel phase synchronization were observed during a seizure event in a short acute recording session. In future work, the real-time seizure detection capability of the SoC will be further validated in vivo with a collection of sufficient seizure activity and objective seizure annotation.

Epileptic Seizure Detection: The classification performance of the SoC was validated on the CHB-MIT EEG and iEEG.org datasets of epileptic patients. We analyzed 983-h EEG recordings of 24 patients and 596-h iEEG recordings of six patients, which contain 176 and 49 annotated seizures, respectively. Blockwise data partitioning was used to avoid data leakage from training to inference. We performed 5-fold cross-validation for most patients and adopted a leave-one-out approach for patients with fewer than five seizures. The number of correctly detected seizures was counted to assess the sensitivity, while the specificity was calculated based on the window-based true negative rate averaged over multiple runs.

In training mode, each patient's multi-channel neural data (18-28 EEG and 47-108 iEEG channels) were fed to the AFE. The digitized AFE outputs were processed offline to extract features using a bit-accurate FEE model in MATLAB for training the classifier. The trained NeuralTree parameters were then stored in the on-chip memory for inference, and the NeuralTree performance on the test data was evaluated. The SoC achieved 95.6%/94% sensitivity and 96.8%/96.9% specificity on the EEG and iEEG datasets, respectively. The SoC's seizure detection performance on an epileptic patient is demonstrated in FIG. 11(e).

Parkinsonian Tremor Detection: The SoC's performance was further validated on a PD patient with rest-state tremor recruited by the University of Oxford. A 4-channel DBS lead was implanted into the subthalamic nucleus to collect LFPs, while the acceleration of the contralateral limb was used to label the tremor. Window-based true positive and negative rates were used to assess the sensitivity and specificity, respectively, using 5-fold cross-validation. The SoC achieved 82.6% sensitivity and 78.4% specificity. FIG. 11(f) presents an example of the SoC's tremor detection performance, where tremor states with inconspicuous neural activity were successfully detected by the NeuralTree. To the best of our knowledge, this is the first demonstration of PD tremor detection with an on-chip classifier.

Other examples of neuro diseases that can be treated by the herein proposed devices, system, and methods are different types of psychiatric disorders such as major depression, anxiety, or PTSD, other movement disorders such as but not limited to tremor, and motor impairments as a result of stroke or spinal cord injury.

### Extension to the PNS and Spinal Cord application domains:

The proposed SoC can also be used in applications other than the central nervous system disorders, such as the peripheral nerve and spinal cord implants for pain control, to treat autoimmune disorders, and for restoring movement in amputees or paralyzed patients. For instance, FIG. 17 shows a bidirectional peripheral nervous system (PNS) implant in the sciatic nerve, where the SoC combined with soft nerve-implanted electrode arrays can perform high-resolution multi-channel electroneurography (ENG) and/or electromyography (EMG) sensing, on-chip feature extraction, machine learning-enabled pain onset detection, and closed-loop neuromodulation for pain relief. The SoC can be similarly used for motor decoding and peripheral nerve stimulation to control muscles or motor prosthetics, coupled with spinal cord stimulation for chronic pain treatment, etc. These fields are currently lacking devices with high-density sensing and closed-loop stimulation capabilities and could greatly benefit from the high selectivity, versatility in biomarker extraction, and intelligent AI-based control mechanism of our SoC.

## Claims

1. An analog front-end device (101) for selectively selecting and reading a plurality of channels from multichannel neural signals input from an electrode array (102), the analog front-end device comprising:
a switch matrix (103) and dual multiplexing choppers (104) for dynamically selecting the plurality of channels from multichannel neural signals input from the electrode array; **characterized in that** the analog front-end device further comprises
a plurality of coarse and a fine DC servo loops (DSL) (200, 201) configured to perform dynamic channel selection by cancelling electrode DC offsets (EDOs) that vary between successive channels, to provide EDO adjusted signals.

2. The analog front-end device of claim 1, wherein the coarse DSL (200) is configured to search for binary bit representations of EDOs from a group of channels, and stores them into a local memory.

3. The analog front-end device of claim 2, wherein the fine DSL (201) is configured to add the stored EDOs and the output of a digital integrator, delta-sigma modulate the added signals, and feeding them back to the input of an amplifier through a digital-to analog converter to remove residual EDOs.

4. A closed-loop neuromodulation system (100), comprising:
an electrode array (102) that is implantable to any one item of a list comprising a brain, a peripheral nervous system, and a spinal cord of a subject;
an analog front-end device (AFD) (101) according to any one of claims 1 to 3;
a finite impulse response (FIR) filter (106) for selectively filtering signals from the AFD;
a feature extraction engine (FEE) (107) operatively connected to the FIR filter, configured to selectively extract features from signals provided by the FIR filter;
a tree-structured hierarchical neural network classifier for detecting disease symptoms; and
a multi-channel stimulator (108) having high-voltage (HV) drivers operatively connectable to the electrode array.

## Patentansprüche

1. Analoge Frontendvorrichtung (101) zum selektiven Auswählen und Lesen einer Vielzahl von Kanälen von neuronalen Mehrkanalsignalen, die von einem Elektrodenarray (102) eingegeben werden, wobei die analoge Frontendvorrichtung Folgendes umfasst:
eine Schaltmatrix (103) und zwei Multiplexing-Chopper (104) zum dynamischen Auswählen der Vielzahl von Kanälen von neuronalen Mehrkanalsignalen, die vom Elektrodenarray eingegeben werden; **dadurch gekennzeichnet, dass** die analoge Frontendvorrichtung ferner Folgendes umfasst
eine Vielzahl von groben und einem feinen DC-Servokreisen (DSL) (200, 201), die dazu ausgelegt sind, durch Abbrechen von DC-Elektrodenversätzen (EDOs), die zwischen aufeinanderfolgenden Kanälen variieren, eine dynamische Kanalauswahl durchzuführen, um angepasste EDO-Signale bereitzustellen.

2. Analoge Frontendvorrichtung nach Anspruch 1, wobei der grobe DSL (200) dazu ausgelegt ist, in einer Gruppe von Kanälen binäre Bitrepräsentationen von EDOs zu suchen, und diese in einem lokalen Speicher speichert.

3. Analoge Frontendvorrichtung nach Anspruch 2, wobei der feine DSL (201) dazu ausgelegt ist, die gespeicherten EDOs und die Ausgabe eines digitalen Integrators hinzuzufügen, an den hinzugefügten Signalen eine Delta-Sigma-Modulation durchzuführen und dieselben über einen Digital-Analog-Wandler zum Eingang eines Verstärkers zurückzuführen, um restliche EDOs zu entfernen.

4. Neuromodulationssystem (100) im geschlossenen Kreis, das Folgendes umfasst:
ein Elektrodenarray (102), das in ein beliebiges Element einer Liste implantierbar ist, die ein Gehirn, ein peripheres Nervensystem und ein Rückenmark eines Patienten umfasst;
eine analoge Frontendvorrichtung (AFD) (101) nach einem der Ansprüche 1 bis 3;
ein endliches Impulsantwort(FIR)-Filter (106) zum selektiven Filtern von Signalen von der AFD;
eine Merkmalsextrahiererungsmaschine (FEE) (107), die an das FIR-Filter wirkgekoppelt und dazu ausgelegt ist, Merkmale von Signalen, die vom FIR-Filter bereitgestellt werden, selektiv zu extrahieren;
einen hierarchischen neuronalen Netzwerkklassifizierer mit Baumstruktur zum Detektieren von Krankheitssymptomen; und
einen mehrkanaligen Stimulator (108), der Hochspannungs(HV)-Treiber aufweist und wirkmäßig mit dem Elektrodenarray verbindbar ist.

## Revendications

1. Dispositif frontal analogique (101) destiné à sélectionner et à lire sélectivement une pluralité de canaux parmi des signaux neuronaux multicanaux entrés via un ensemble d'électrodes (102), le dispositif frontal analogique comprenant :
une matrice de commutation (103) et des hacheurs à multiplexage double (104) pour sélectionner de manière dynamique la pluralité de canaux parmi les signaux neuronaux multicanaux entrés via l'ensemble d'électrodes ; **caractérisé en ce que** le dispositif frontal analogique comprend en outre
une pluralité de boucles d'asservissement CC [courant continu] (DSL) grossières et fines (200, 201), configurées pour effectuer une sélection de canal dynamique en annulant les décalages CC d'électrode (EDO) qui varient entre les canaux successifs, afin de fournir des signaux EDO ajustés.

2. Dispositif frontal analogique selon la revendication 1, dans lequel la DSL grossière (200) est configurée pour rechercher des représentations de bit binaires des EDO dans un groupe de canaux et les stocker dans une mémoire locale.

3. Dispositif frontal analogique selon la revendication 2, dans lequel la DSL fine (201) est configurée pour additionner les EDO stockés et la sortie d'un intégrateur numérique, moduler en delta-sigma les signaux additionnés, et les renvoyer à l'entrée d'un amplificateur via un convertisseur numérique-analogique afin d'éliminer les EDO résiduels.

4. Système de neuromodulation en boucle fermée (100), comprenant :
un ensemble d'électrodes (102) implantable dans l'un quelconque des éléments d'une liste comprenant un cerveau, un système nerveux périphérique et une moelle spinale d'un sujet ;
un dispositif frontal analogique (AFD) (101) selon l'une quelconque des revendications 1 à 3 ;
un filtre à réponse impulsionnelle finie (FIR) (106) pour filtrer sélectivement les signaux provenant de l'AFD ;
un moteur d'extraction de caractéristiques (FEE) (107) connecté fonctionnellement au filtre FIR, configuré pour extraire sélectivement des caractéristiques à partir des signaux produits par le filtre FIR ;
un classificateur de réseau neuronal hiérarchique à structure arborescente pour détecter des symptômes de maladie ; et
un stimulateur multicanal (108) ayant des pilotes haute tension (HV) connectables fonctionnellement à l'ensemble d'électrodes.
